# EUROPEAN PATENT APPLICATION

(11) **EP 4 257 121 A1**
(43) Date of publication of application: **11.10.2023**
(21) Application number: 21914624.8
(22) Date of filing: 30.12.2021
(51) Int. Cl.: A61K 9/19, A61K 31/704, A61K 47/10, A61K 47/12, A61K 47/22, A61K 47/26, A61K 47/40, A61K 47/42, A61K 45/06, A61K 31/437, A61K 31/7048, A61K 31/136, A61K 9/08, A61P 13/10, A61P 35/00, A61P 35/04, A61K 31/4745

(54) **BLADDER PERFUSION PHARMACEUTICAL COMPOSITION, PREPARATION METHOD THEREFOR AND APPLICATION THEREOF**

(30) Priority: 30.12.2020 CN 202011609565; 09.12.2021 CN 202111498069; 09.12.2021 CN 202111499923
(71) Applicant: Innobm Pharmaceuticals Co., Ltd., Suzhou, Jiangsu 215123 (CN)
(72) Inventor: LIU, Zhuang, Suzhou, Jiangsu 215123 (CN); DENG, Zhongqing, Suzhou, Jiangsu 215123 (CN); XU, Huan, Suzhou, Jiangsu 215123 (CN); TAO, Huiquan, Suzhou, Jiangsu 215123 (CN); WU, Yuchen, Suzhou, Jiangsu 215123 (CN); ZHOU, Xuanfang, Suzhou, Jiangsu 215123 (CN); LI, Guangzhi, Suzhou, Jiangsu 215123 (CN); WU, Song, Suzhou, Jiangsu 215123 (CN)
(74) Representative: Sun, Yiming
(86) International application number: PCT/CN2021/143056
(87) International publication number: WO 2022/143893

(57) **Abstract**

The present application relates to a bladder perfusion pharmaceutical composition, including an immunological adjuvant or a soluble salt thereof, and a chemical drug capable of causing immunogenic cell death. When the immunological adjuvant and the soluble salt thereof and the chemical drug capable of causing immunogenic cell death are used in combination as bladder perfusion drugs, the side effect of the chemical drug can be reduced, a synergistic anti-cancer effect can be produced, and the probability of cancer metastasis and recurrence can be reduced. The present application further provides a method for preparing the bladder perfusion pharmaceutical composition, and a use thereof.

## Description

### FIELD OF THE INVENTION

The present application relates to the field of biomedicine, in particular to a bladder perfusion pharmaceutical composition, a preparation method therefor and use thereof.

### BACKGROUND TECHNIQUE

Bladder cancer is a common tumor of urinary system. Although it has a lower incidence and mortality compared with lung cancer and gastrointestinal tumor, its high recurrence rate has made bladder cancer one of the most expensive cancers in clinical treatment. Perfusion chemotherapy after transurethral resection of bladder cancer tumor is the first choice for the treatment of bladder cancer. But currently, perfusion treatment has the disadvantages of low clinical absolute remission rate, high tumor recurrence rate, great toxic side effects and poor prognosis of patients. Meanwhile, the incidence of bladder cancer in China has been increasing year by year in recent years, which is higher than the world's average. In addition, the research on novel perfusion small molecule drugs has been slow in recent 30 years, and severe problems in the clinical prevention and treatment of bladder cancer are faced in our country.

There are two common types of bladder cancer clinically, non-muscular invasive bladder cancer (NMIBC) and muscular invasive bladder cancer (MIBC). Among those bladder tumor clinical therapeutics, transurethral resection of bladder tumor (TURBT) accompanied by drug perfusion treatment is the first-line standard therapeutic regimen for non-muscular invasive bladder cancer. Studies have shown that patients with non-muscular invasive bladder cancer have a recurrence probability of 50% to 70% within 5 years after TURBT bladder tumor resection. Bladder perfusion directly kills tumor cells or induces nonspecific immune response in vivo by injecting tumor therapeutic formulations such as chemotherapeutic drugs into the bladder after TURBT bladder tumor resection, so as to achieve the effect of inhibiting the metastasis and recurrence of bladder cancer. But currently, bladder perfusion treatment has the disadvantages of low absolute remission rate, higher tumor recurrence rate, and great toxic side effects and poor prognosis of patients clinically. Studies have shown that some chemotherapeutic drugs can induce the expression of immunogenic protein molecules on cell surfaces by inducing apoptosis or other programmed death of tumor cells, and then stimulate the anti-tumor immune response of organisms, which phenomenon is called immunogenic cell death (ICD). Therefore, it may be one of the ways to prepare and improve the drug system of chemotherapy-immunization combined treatment. Currently, only a few clinically relevant drugs have been proved to be capable of initiating the ICD phenomenon in clinical application. These drugs mainly include: (1) doxorubicin and anthracycline-based drugs, which have been used to treat cancers such as small cell lung cancer; (2) epirubicin, an anthracycline-based drug allowed for breast cancer patients; (3) idarubicin, an anthracycline-based drug currently used to treat acute myelocytic leukemia; (4) mitoxantrone, an anthracycline-based drug approved for patients with breast cancer, non-Hodgkin's lymphoma and prostate cancer; (5) bleomycin, a glycopeptide antibiotic used for the treatment of testicular cancer and palliative treatment of squamous carcinoma of head and neck, cervix and vulva; (6) VELCADE (bortezomib), a proteasome inhibitor for patients with multiple myeloma and T-cell lymphoma; and (7) oxaliplatin used in combination with 5-fluorouracil and folinic acid for the treatment of advanced colorectal cancer. However, currently, it is found that most ICD drugs cannot play an immune killing-promoting role against tumor cells effectively and sustainably, because immune cells cannot effectively identify and kill cancer cells as long as there are tumor-associated antigens. It is the killer T cells in immune cells that really play a role and can pursue and destroy cancer cells. The so-called killer T cells are activated T cells. T cells need antigen presenting cells (APC) to ingest, process and present these antigens to themselves before they can be activated and really play the effect, while antigen presenting cells need the help of immunological adjuvants to present antigens more effectively.

Anthracycline drugs are highly susceptible to a plurality of transitional epithelial cell cancers. Epirubicin is a third-generation anthracycline-based semi-synthetic compound, which can be directly embedded between DNA base pairs, interfere with transcription process, prevent the formation of mRNA and play an anti-tumor role. Currently, anthracycline-based chemotherapeutic drugs represented by epirubicin and pirarubicin are the first-line treatment drugs for perfusion treatment of bladder cancer. However, for high-risk patients with non-muscular invasive bladder cancer, the tumor still has a high recurrence rate (more than 50%) within one year after bladder perfusion treatment by administration of epirubicin or pirarubicin.

In recent years, immunotherapy of tumors is of particular concern. The key steps of immunotherapy are sufficient antigen exposure, effective antigen presentation and subsequent invasion and killing in tumors. Immunoagonists, as key steps connecting the preceding with the following, can achieve antigen presentation. Among them, representative immunoagonists, such as Toll-like receptors (TLRs), are pattern recognition receptors. As the important constituent part of the innate immune system, Toll-like receptors have the functions of perceiving and recognizing pathogen-associated molecular patterns (PAMPs). The receptor is widely expressed in immune cells, which will promote T cell responses against tumors after being stimulated accordingly. For example, imiquimod (R837) and immunostimulant oligonucleotide CpG recognize and stimulate TLR7 and TLR9 respectively, and the synthesized immunomodulator R848 (Resiquimod) can activate TLR7 and TLR8. A large number of studies have reported that TLR agonists have the function of immunological adjuvants and have great potential for tumor treatment, but their effectiveness and applicability are limited by the side effects of systemic release of pro-inflammatory factors and cytokines. Therefore, the in vivo treatment with TLR7 as ligand mainly focuses on the application with TLR7 as immunological adjuvant target, such as antiviral or antitumor local treatment.

In clinical trials, there are studies on the use of TLR agonists in the treatment of bladder cancer. For example, TMX-101 directly uses the gel formulation containing R837 component for bladder perfusion. It was reported that R837 showed good safety in this clinical trial, and some patients benefited. After being acquired, the project continued to carry out research for the combined treatment of bladder cancer with monoclonal antibodies, and the latest research results have not been disclosed yet. These clinical trials suggest the application potential of TLR agonists in bladder perfusion treatment, but all of them lack the process of antigen exposure, thus they cannot fully exert the immune synergistic effect of TLR agonists, and there is room for further optimization.

Simple perfusion chemotherapy has poor inhibitory effect on the progress of bladder cancer, and it is prone to produce toxic and side effects and adverse reactions, and easily tolerated by patients and the prognosis is not ideal. How to improve the therapeutic effect of bladder perfusion chemotherapy is a major subject in the technology of bladder perfusion treatment. How to combine water soluble chemotherapy drugs with lipid soluble immunological adjuvants is also a key issue to further promote the chemotherapy-immunization combined treatment of bladder cancer.

### SUMMARY OF THE INVENTION

The present application provides a bladder perfusion pharmaceutical composition, which can produce a synergistic anticancer effect, reduce the probability of cancer metastasis and recurrence, and also inhibit the growth of distant metastatic tumors and reduce the probability of tumor recurrence through immune responses while effectively killing tumor in-situ.

In one aspect, the present application provides a bladder perfusion pharmaceutical composition, including an immunological adjuvant or a soluble salt thereof, and a chemotherapy drug capable of causing immunogenic cell death.

In certain embodiments, the chemotherapy drug capable of causing immunogenic cell death includes: an anthracycline-based chemotherapeutic drug, a platinum-based chemotherapeutic drug, fluorouracil or gemcitabine.

In certain embodiments, the anthracycline-based chemotherapy drug includes epirubicin or a soluble salt thereof, pirarubicin or a soluble salt thereof, mitoxantrone or a soluble salt thereof, doxorubicin or a soluble salt thereof, aclarubicin or a soluble salt thereof, and idarubicin or a soluble salt thereof.

In certain embodiments, the soluble salt is hydrochloride.

In certain embodiments, the anthracycline-based chemotherapeutic drug includes doxorubicin hydrochloride, epirubicin hydrochloride, pirarubicin hydrochloride, and mitoxantrone hydrochloride.

In certain embodiments, the platinum-based chemotherapeutic drug includes nedaplatin, carboplatin, lobaplatin, or oxaliplatin.

In certain embodiments, the immunological adjuvant or the soluble salt thereof includes a soluble salt of the immunological adjuvant.

In certain embodiments, the immunological adjuvant or the soluble salt thereof includes an imidazoquinoline-based immunological adjuvant or a soluble salt thereof.

In certain embodiments, the imidazoquinoline-based immunological adjuvant includes imiquimod and a derivative thereof, or resiquimod and a derivative thereof, or a soluble salt of imiquimod and the derivative thereof, or a soluble salt of resiquimod and the derivative thereof.

In certain embodiments, the soluble salt of the immunological adjuvant includes at least one of imiquimod (also known as R837) hydrochloride, resiquimod (also known as R848) hydrochloride or other pharmaceutically acceptable salts, CpG, polyIC, polyICLC and a STING stimulator.

In certain embodiments, the mass ratio of the immunological adjuvant or the soluble salt thereof to the chemotherapy drug capable of causing immunogenic cell death is 1:100 to 6:1.

In certain embodiments, the mass ratio of the imiquimod R837 hydrochloride to the chemotherapy drug capable of causing immunogenic cell death is 1:20 to 1:1, and the chemotherapy drug capable of causing immunogenic cell death is fluorouracil or gemcitabine.

In certain embodiments, the mass ratio of the imiquimod R837 hydrochloride to the chemotherapy drug capable of causing immunogenic cell death is 1:1 to 6:1, wherein the chemotherapy drug includes an anthracycline-based chemotherapeutic drug or a platinum-based chemotherapeutic drug, and the anthracycline-based chemotherapeutic drug includes doxorubicin hydrochloride, epirubicin hydrochloride, pirarubicin hydrochloride, and mitoxantrone hydrochloride; and the platinum-based chemotherapeutic drug includes nedaplatin, carboplatin, lobaplatin, or oxaliplatin.

In certain embodiments, the mass ratio of the resiquimod R848 hydrochloride to the chemotherapy drug capable of causing immunogenic cell death is 1:20 to 1:1, wherein the chemotherapy drug capable of causing immunogenic cell death includes an anthracycline-based chemotherapeutic drug or a platinum-based chemotherapeutic drug, and the anthracycline-based chemotherapeutic drug includes doxorubicin hydrochloride, epirubicin hydrochloride, pirarubicin hydrochloride or mitoxantrone hydrochloride; and the platinum-based chemotherapeutic drug includes nedaplatin, carboplatin, lobaplatin, or oxaliplatin.

In certain embodiments, the mass ratio of the resiquimod R848 hydrochloride to the chemotherapy drug capable of causing immunogenic cell death is 1:100 to 1:10, wherein the chemotherapy drug capable of causing immunogenic cell death includes fluorouracil or gemcitabine.

In certain embodiments, the concentration of the immunological adjuvant ranges from 0.5 mg/mL to 30 mg/mL.

In certain embodiments, the bladder perfusion pharmaceutical composition further includes a pH regulator.

In certain embodiments, after the bladder perfusion pharmaceutical composition is reconstituted, the pH regulator enables the pH of the reconstituted solution to be 3.8 to 5.5 when the concentration of the anthracycline-based chemotherapy drug is 1 to 5 mg/mL.

In certain embodiments, the bladder perfusion pharmaceutical composition further includes a lyoprotectant.

In certain embodiments, the lyoprotectant includes at least one of sucrose, lactose, mannitol and cyclodextrin.

In certain embodiments, the lyoprotectant is lactose.

In certain embodiments, the bladder perfusion pharmaceutical composition is a lyophilized powder formulation.

In certain embodiments, the mass ratio of the anthracycline-based chemotherapy drug to the immunological adjuvant is 1:0.1 to 1:10.

In certain embodiments, the mass ratio of the anthracycline-based chemotherapy drug to imiquimod and the derivative thereof is 1:1 to 1:10.

In certain embodiments, the mass ratio of the anthracycline-based chemotherapy drug to the soluble salt of imiquimod and the derivative thereof is 1:1 to 1:10.

In certain embodiments, the mass ratio of the anthracycline-based chemotherapy drug to resiquimod is 1:0.1 to 1:5.

In certain embodiments, the mass ratio of the anthracycline-based chemotherapy drug to the soluble salt of resiquimod and the derivative thereof is 1:0.1 to 1:5.

In certain embodiments, the bladder perfusion pharmaceutical composition includes: epirubicin or a soluble salt thereof, imiquimod or a soluble salt thereof, and a pH regulator.

In certain embodiments, the bladder perfusion pharmaceutical composition is a lyophilized powder formulation.

In certain embodiments, the bladder perfusion pharmaceutical composition further includes a lyoprotectant.

In certain embodiments, the pH of the lyophilized powder dosage form after reconstitution is 3.8 to 5.5 when the concentration of epirubicin is 1 to 5 mg/mL.

In certain embodiments, the pH of the lyophilized powder dosage form after reconstitution is 4.0 to 5.0 when the concentration of epirubicin is 1 to 5 mg/mL.

In certain embodiments, the pH of the lyophilized powder dosage form after reconstitution is 4.0 to 4.2, 4.2 to 4.5 or 4.5 to 5.0 when the concentration of epirubicin is 1 to 5 mg/mL.

In certain embodiments, the mass ratio of the epirubicin or the hydrochloride thereof to imiquimod or the soluble salt thereof is 1:1 to 1:10.

In certain embodiments, the mass ratio of the epirubicin or the hydrochloride thereof to imiquimod or the soluble salt thereof is 1:2 to 1:4.

In certain embodiments, the lyoprotectant includes sucrose, lactose, mannitol, and cyclodextrin.

In certain embodiments, the lyoprotectant is lactose.

In certain embodiments, the mass fraction of the lyoprotectant in the lyophilized powder formulation is 65% to 96%.

In certain embodiments, the content of the lyoprotectant in the lyophilized powder formulation is 80% to 94%.

In certain embodiments, the bladder perfusion pharmaceutical composition further includes a mucosal penetration enhancer.

In certain embodiments, the mucosal penetration enhancer includes at least one of azone, hyaluronidase, lauryl alcohol, and oleic acid.

In another aspect, the present application further provides a method for preparing a bladder perfusion pharmaceutical composition, including the following steps: S1: dissolving an immunological adjuvant with a dilute acid to obtain a dilute acid salt solution of the immunological adjuvant; S2: dissolving a chemotherapy drug capable of causing immunogenic cell death with the dilute acid salt solution obtained in S1 to obtain a mixed solution; and S3: adding a pH regulator into the mixed solution in S1, and controlling the pH between 2.0 and 5.5.

In certain embodiments, the step S3 further includes the following steps: S31: adding a lyoprotectant into the mixed solution; and/or S32: adding a mucosal penetration enhancer into the mixed solution.

In certain embodiments, the preparation method further includes step S4: lyophilizing the final solution.

In certain embodiments, the pH is controlled between 3.8 and 5.5 in the step S3.

In certain embodiments, in the preparation method, the chemotherapy drug capable of causing immunogenic cell death includes an anthracycline-based chemotherapy drug, and the final concentration of the anthracycline-based chemotherapy drug in step S3 is 1 to 5 mg/mL.

In another aspect, the present application further provides a method for preparing a bladder perfusion pharmaceutical composition, including the following steps: S1: adding immunological adjuvants CpG, polyIC, polyICLC, and a water-soluble STING stimulator as well as a chemotherapy drug capable of causing immunogenic cell death into water for injection, and uniformly mixing until dissolution; and S2: adding a suitable lyoprotectant into the solution in S1, and lyophilizing the solution.

In another aspect, the present application further provides use of the bladder perfusion pharmaceutical composition in preparation of a bladder perfusion formulation.

Persons skilled in the art can recognize other aspects and advantages of their application from the following detailed description. The following detailed description only shows and indicates exemplary embodiments of this application. As those skilled in the art will appreciate, the disclosure of this application enables persons skilled in the art to modify the disclosed embodiments without departing from the spirit and scope of the invention involved by this application. Correspondingly, the drawings and the description in the specification of this application are only exemplary, and not restrictive.

### BRIEF DESCRIPTION OF THE DRAWINGS

The specific features of the invention involved by this application are as shown in the appended claims. By reference to the exemplary embodiments detailed below and the accompanying drawings, the characteristics and advantages of the invention involved in this application can be better understood. The drawings are briefly described as follows:
Fig. 1 is an ultrasound image of bladder sites at different time after combined perfusion of doxorubicin hydrochloride (DOX) and imiquimod hydrochloride (R837) for the treatment of bladder in situ tumor;
Fig. 2 is a physical picture of the final sizes of different groups of tumors in the experiment of combined perfusion of pirarubicin hydrochloride (THP) and imiquimod hydrochloride (R837) for the treatment of bladder in situ tumor;
Fig. 3 is an in vivo fluorescence image of mice at different time points in the experiment of combined perfusion of pirarubicin hydrochloride (THP) and imiquimod hydrochloride (R837) in different ratios for the treatment of bladder in situ tumor;
Fig. 4 is a statistical graph of the mean fluorescence intensities of the bladder sites of mice at different time points in the experiment of combined perfusion of pirarubicin hydrochloride (THP) and imiquimod hydrochloride (R837) in different ratios for the treatment of bladder in situ tumor;
Fig. 5 is a tumor fluorescence image in the experiment of combined perfusion of epirubicin hydrochloride (EPI) and imiquimod hydrochloride (R837) for the treatment of bladder in situ tumor;
Fig. 6 is a statistical data graph of the fluorescence intensities of tumor sites at the end of the combined perfusion of epirubicin hydrochloride (EPI) and imiquimod hydrochloride for the treatment of bladder in situ tumor;
Fig. 7 is a graph of the tumor growth in mice which were subjected to perfusion treatment of bladder cancer by using EPI and R837 in combination;
Fig. 8 is a graph showing changes in the body weights of mice within 1-9 days after perfusion administration of EPI and R837 in combination in the toxicity experiment (the dosage ratio is 1:6);
Fig. 9 is a graph showing changes in the body weights of mice within 1-4 days after perfusion administration of EPI and R837 in combination in the toxicity experiment (the dosage ratio is 1:10);
Fig. 10 is a statistical graph of the EPI contents in the blood of mice;
Fig. 11 is a statistical graph of the fluorescence intensities of the EPI contents in the bladder tissues of mice;
Fig. 12 is a statistical graph of the intensities of the EPI fluorescence signals in the macrophages in the bladder tissues of mice;
Fig. 13 is a statistical graph of the tumor mass of mice with bladder cancer in different groups after a mixed lyophilized powder formulation of epirubicin and imiquimod was applied to the mice to treat bladder cancer by bladder perfusion;
Fig. 14 is a statistical graph of the tumor mass of mice after a mixed lyophilized powder formulation of pirarubicin and imiquimod was used to treat bladder cancer in the mice by perfusion;
Fig. 15 is a statistical graph of the tumor mass of mice in different groups after a mixed lyophilized powder formulation of doxorubicin and imiquimod was used to treat bladder cancer by bladder perfusion;
Fig. 16 is an anatomical image of the focus sites of mice in different groups on day 3 after the second administration of bladder perfusion treatment;
Fig. 17 is a statistical graph of the tissue mass in the focus sites of mice in different groups on day 3 after the second administration of bladder perfusion treatment;
Fig. 18 is a statistical graph of the flow analysis of killer T cells invaded in the tumor sites of mice in different groups on day 3 after the second administration of bladder perfusion treatment;
Fig. 19 is a statistical graph of the flow analysis of CD3+/CD4+T cells in the tumor sites of mice in different groups on day 3 after the second administration of bladder perfusion treatment;
Fig. 20 is a statistical graph of the IFN-γ contents in CD8+ cells in the tumor sites of mice in different groups after being stimulated by a cell stimulating mixture on day 3 after the second administration of bladder perfusion treatment;
Fig. 21 is a statistical graph of the relative contents of tumor necrosis factor in the focus sites of mice in different groups on day 3 after the second administration of bladder perfusion treatment;
Fig. 22 is a statistical graph of the relative contents of γ interferon in the focus sites of mice in different groups on day 3 after the second administration of bladder perfusion treatment;
Fig. 23 is a statistical graph of the effect of a mucosal penetration enhancer on the retention of epirubicin in bladders;
Fig. 24 is a statistical graph of the effect of the mucosal penetration enhancers on the retention of imiquimod in bladders.

### DETAILED DESCRIPTION OF THE INVENTION

The implementation of this application will be illustrated in the following specific examples. Persons skilled in the art can readily understand other advantages and effects of this application from the disclosure of the specification.

To solve the related technical problems, the present application provides a bladder perfusion pharmaceutical composition. Through experiments, our research and development team has found that when a soluble salt of an immunological adjuvant and a chemotherapy drug capable of causing immunogenic cell death (also known as ICD chemotherapy drug) are used in combination as bladder perfusion drugs, an anti-cancer pharmaceutical composition is produced, by which a synergistic anticancer effect is produced, and the probability of cancer metastasis and recurrence is reduced. Because of the anti-cancer pharmaceutical composition, the tumor in-situ can be effectively killed, and additionally, the growth of distant metastatic tumors can be inhibited, and the probability of tumor recurrence can be reduced through immune responses. Also, the attenuation effect of the system of the invention is different from the previous mechanism of reducing the toxicity of the drug system by changing the local and systemic absorption of the perfused drugs through dosage forms. The invention significantly reduces the toxic and side effects of chemotherapeutic drugs on normal tissues by perfusion administration of a drug combination, and based on exerting the immunogenicity of the chemotherapy drugs, making the drug combination enter the focus through the channel that enhances the phagocytosis of macrophages on the tumor extracellular free chemotherapeutic drugs in the tissues. This drug entry mode was first attempted and observed in perfusion studies.

The invention provides a bladder perfusion pharmaceutical composition, including: a soluble salt of an immunological adjuvant, and a chemotherapy drug capable of causing immunogenic cell death. Further, the mass ratio of the soluble salt of the immunological adjuvant to the chemotherapy drug capable of causing immunogenic cell death is 1:100 to 6:1.

Further, the mass ratio of the soluble salt of the immunological adjuvant to the chemotherapy drug capable of causing immunogenic cell death is 2:1 to 4:1.

Further, the chemotherapy drug capable of causing immunogenic cell death includes an anthracycline-based chemotherapeutic drug, a platinum-based chemotherapeutic drug, fluorouracil, or gemcitabine; optionally, the anthracycline-based chemotherapeutic drug includes doxorubicin hydrochloride, epirubicin hydrochloride, pirarubicin hydrochloride, and mitoxantrone hydrochloride; and the platinum-based chemotherapeutic drug includes nedaplatin, carboplatin, lobaplatin, or oxaliplatin.

Further, the soluble salt of the immunological adjuvant includes at least one of imiquimod R837 hydrochloride or other pharmaceutically acceptable soluble salts, resiquimod R848 hydrochloride or other pharmaceutically acceptable soluble salts of immunological adjuvants, CpG, polyIC, polyICLC, and a STING stimulator (which is and can include IMSA-101, GSK-3745417, BMS-986301, SB-11285 and MK-1454).

Further, the mass ratio of the imiquimod R837 hydrochloride to the chemotherapy drug capable of causing immunogenic cell death is 1:20 to 1:1, and the chemotherapy drug capable of causing immunogenic cell death is fluorouracil or gemcitabine.

Further, the mass ratio of the imiquimod R837 hydrochloride to the chemotherapy drug capable of causing immunogenic cell death is 1:1 to 6:1, wherein the chemotherapy drug includes an anthracycline-based chemotherapeutic drug or a platinum-based chemotherapeutic drug, and the anthracycline-based chemotherapeutic drug includes doxorubicin hydrochloride, epirubicin hydrochloride, pirarubicin hydrochloride, and mitoxantrone hydrochloride; and the platinum-based chemotherapeutic drug includes nedaplatin, carboplatin, lobaplatin, or oxaliplatin.

Further, the mass ratio of the resiquimod (R848) hydrochloride to the chemotherapy drug capable of causing immunogenic cell death is 1:10 to 1:1, wherein the chemotherapy drug capable of causing immunogenic cell death includes an anthracycline-based chemotherapeutic drug or a platinum-based chemotherapeutic drug, and the anthracycline-based chemotherapeutic drug includes doxorubicin hydrochloride, epirubicin hydrochloride, pirarubicin hydrochloride, and mitoxantrone hydrochloride; and the platinum-based chemotherapeutic drug includes nedaplatin, carboplatin, lobaplatin, or oxaliplatin.

Further, the mass ratio of the resiquimod (R848) hydrochloride to the chemotherapy drug capable of causing immunogenic cell death is 1:100 to 1:10, wherein the chemotherapy drug capable of causing immunogenic cell death includes fluorouracil or gemcitabine.

Further, it also includes a lyophilization cosolvent (such as mannitol, lactose, etc.) and a pH regulator.

Further, the concentration of the soluble salt of the immunological adjuvant ranges from 0.5 mg/mL to 30 mg/mL.

Further, the dosage form is lyophilized powder.

The invention further provides a method for preparing a bladder perfusion pharmaceutical composition, including the following steps:
Scheme 1:
   S1: dissolving the immunological adjuvant imiquimod (R837) or resiquimod (R848) in a dilute acid solution (such as hydrochloric acid, lactic acid, and acetic acid) or other pharmaceutically acceptable soluble salt solutions of immunological adjuvants (such as glucopyranoside lipid A (MPLA)), adding a chemotherapy drug capable of causing immunogenic cell death into the solution, and uniformly mixing until dissolution;
   S2: adding a suitable lyophilization cosolvent (such as mannitol, lactose, etc.) and a pH regulator into the solution in S1, and controlling the pH value between 2.0 and 5.5;
   and S3: lyophilizing the solution obtained in the step S2.
Scheme 2:
   A method for preparing a bladder perfusion pharmaceutical composition, characterized by experiment including the following steps:
   S1: adding the immunological adjuvants CpG, polyIC, polyICLC, and a water soluble STING stimulator as well as a chemotherapy drug capable of causing immunogenic cell death into water for injection, and uniformly mixing until dissolution;
   and S2: adding a suitable lyophilization cosolvent (such as mannitol, lactose, etc.) into the solution in S1, and lyophilizing the solution.

The invention further provides the application of the combination drug in preparing bladder perfusion drugs.

The technical scheme of the invention has the following beneficial technical effects:
The systemic toxic and side effects of ICD chemotherapy drugs can be reduced. According to the available evidence, it is preliminarily inferred that the mechanism of this phenomenon is that the combined immunological adjuvant enhances the phagocytosis of macrophages on tumor extracellular free chemotherapeutic drugs in tissues, thus significantly reducing the toxic side effects of the chemotherapeutic drugs on normal tissues. By perfusion treatment after transurethral resection of bladder cancer tumor, ICD drugs can initiate immunogenic cell death of tumor cells, thus activating the immune system to specifically eliminate cancer cells. Antigen presenting cells can guide the immune system to track, identify and kill other cancer cells after phagocytosing dead cancer cells. Therefore, after ICD chemotherapeutic drugs cause the immunogenic death of cancer cells, tumor-associated antigens in cancer cell residues will be exposed to immune cells, thereby providing targets to help immune cells identify cancer cells and helping the immune system establish a tumor cell-specific immune response. After being recognized by TLR, the soluble immunological adjuvant activates a series of downstream signals, induces the secretion of inflammatory cytokines, chemokines and type I interferon, and promotes tumor-associated antigens to be presented to T cells with a tumor killing effect by antigen presenting cells more effectively. Therefore, after ICD chemotherapeutic drugs cause cancer cells to die and become tumor-associated antigens, the introduction of immunological adjuvants can further stimulate antigen-presenting cells to uptake and process these antigens and present them to T cells more effectively, thus amplifying this anti-tumor immune response. Also, the existence of immunological adjuvants also promotes the enrichment of a large number of macrophages around the tumor tissues of the bladder. After ICD drugs play the role of initial chemotherapy and immune stimulation, the macrophages phagocytosed a large number of free chemotherapeutic drugs outside tumor cells, which can reduce the damage of the chemotherapeutic drugs to normal tissues, thus reducing the systemic toxic and side effects of the ICD chemotherapy drugs, as well as increasing the dosage of ICD drugs in a certain range or improving the overall therapeutic effect.

Local combined administration of soluble immunological adjuvants and ICD chemotherapeutic drugs can significantly enhance the effect of the chemotherapeutic drugs on tumor treatment and prevention of metastasis and recurrence. After ICD chemotherapeutic drugs cause cancer cells to die and become tumor-associated antigens, the introduction of immunological adjuvants can further stimulate APC to uptake and process these antigens and present them to T cells more effectively, thus amplifying this anti-tumor immune response. Combined perfusion application of immunological adjuvants and ICD chemotherapeutic drugs has an amelioration effect on the treatment and prevention of recurrence of bladder cancer. Just like a tumor vaccine produced in vivo, under the combined action of ICD chemotherapeutic drugs and immunological adjuvants, the in-situ tumor is killed and turned into a tumor vaccine. Therefore, the drug system of ICD chemotherapeutic drugs and immunological adjuvants used in combination as described in the invention has the technical effect of efficiently treating and preventing bladder cancer metastasis and recurrence.

The applicant attempted to use an anthracycline-based chemotherapy drug and imiquimod in combination, and apply them to perfusion treatment of bladder cancer. After directly mixing the two drugs, bladder perfusion was carried out to verify the therapeutic effect. The results have shown that, taking the chemotherapeutic drug epirubicin and imiquimod as an example, when the ratio of the chemotherapeutic drug epirubicin to imiquimod is in a suitable range, the therapeutic effect can be significantly enhanced compared with that of the chemotherapeutic drug alone, and the toxic side effects are not increased. It is expected to become a novel formulation of bladder perfusion drugs with significant clinical value. Further study has shown that the anti-tumor mechanism of this novel formulation is mainly that tumor-related antigens are exposed through inducing the immunogenic death of tumor cells by chemotherapeutic drugs such as epirubicin. Also, imiquimod, as a TLR7 agonist, can activate the immune system, recruit antigen-presenting cells into tumors to recognize the tumor antigens, and then activate T cell immune responses against tumors. Based on this principle, additionally, we attempted the effects of using different anthracycline-based chemotherapy drugs and imiquimod (R837) or resiquimod (R848) in combination to treat tumors, and all of them achieved ideal therapeutic effects.

However, in the further study of the formulation, we have found that the existence of imiquimod molecules in the simple mixed formulation of an anthracycline-based chemotherapeutic drug such as epirubicin and the immunological adjuvant imiquimod will lead to the instability of the molecular structure of the anthracycline-based chemotherapeutic drug such as epirubicin. Whether stored in the form of injection or lyophilized powder after mixing, the impurity content in the formulation will increase obviously, and break through the impurity limits of the drugs specified in Pharmacopoeia within 5 days. Thus it can't be a stable pharmaceutical formulation which is convenient for clinical application, and the simple mixing of them is not benefit for the chemical stabilities of the drug molecules. Therefore, how to enhance the chemical stability of the compound formulation of anthracycline-based chemotherapy drugs and imidazoquinoline-based immunomodulators is the key problem in designing and preparing the compound formulation for using the two in combination. To solve the problems derived from above, we further systematically studied the stability of the compound formulation of anthracycline-based chemotherapy drugs and imidazoquinoline-based immunomodulators.

First of all, we have found that epirubicin and imiquimod are more stable under the condition of a low pH value (such as pH 3.0) in solution. However, the anthracycline-based chemotherapy drug will still degrade gradually, and the mixed formulation under this condition cannot be stored for a long time. Thus the injection dosage form is not feasible for this formulation. For lyophilized formulations, we have unexpectedly found that among the common lyoprotectants, lactose has a significant protective effect on the stability of the formulation, and the increase of lactose content will also be favorable to enhancing the stabilities of the formulations. Nevertheless, the lyophilized formulation prepared under the condition of pH 3.0 cannot be stored for a long time, and it will not pass the inspection of the accelerated stability experiment. We have found that this lyophilized compound formulation of anthracycline-based chemotherapy drugs and immunological adjuvants has a stable "golden pH value interval", and the epirubicin/imiquimod compound formulation prepared in this pH range has a very good stability! This phenomenon is contrary to the conventional understanding of the epirubicin single formulation by those skilled in the art (conventionally, the lower the pH, the more stable it is) and our prediction of the stability trend of the compound formulation in solution (the lower the pH, the more stable it is).

Furthermore, we investigated the stability of the compound lyophilized powder formulations of other anthracycline-based drugs such as mitoxantrone and doxorubicin, and imidazoquinoline-based immunomodulators (R837 or R848). Based on the detailed investigation results of epirubicin and R837, we attempted the stabilities of different compounds under similar pH conditions. The results have shown that the chemical structures of these compounds are similar or the same, which is verified as the best formula of the compound formulation that makes any combination of the two substances have better stability.

The invention provides a compound formulation of bladder perfusion drugs, including an anthracycline-based chemotherapy drug, an immunomodulator, and a pH regulator, wherein the immunomodulator includes imidazoquinoline-based immunomodulators and soluble salts thereof.

Further, the anthracycline-based chemotherapy drug includes epirubicin or a soluble salt thereof, pirarubicin or a soluble salt thereof, mitoxantrone or a soluble salt thereof, doxorubicin or a soluble salt thereof, aclarubicin or a soluble salt thereof, and idarubicin or a soluble salt thereof.

Further optionally, the soluble salt is hydrochloride.

Further, the lyophilized powder formulation of the bladder perfusion drugs further includes a lyoprotectant.

Further, the lyoprotectant includes sucrose, lactose, mannitol, and cyclodextrin.

Further optionally, the lyoprotectant is lactose.

Further, the mass fraction of the lyoprotectant in the lyophilized powder formulation is 65% to 96%.

Optionally, the content of the lyoprotectant in the lyophilized powder formulation is 80% to 94%.

Further, the pH regulator is a weak alkali, an alkaline buffer, sodium hydroxide and hydrochloric acid.

Further optionally, the pH regulator is sodium bicarbonate and hydrochloric acid.

Further, after the compound formulation of the bladder perfusion drugs is reconstituted, the pH of the solution is 3.8 to 5.5 when the concentration of the anthracycline-based chemotherapy drug is 1 to 5 mg/mL. It should be noted that the purpose of defining the reconstitution concentration here is to define the range of the pH value after reconstitution, and is not to limit the reconstitution concentration.

Further optionally, after the lyophilized powder formulation of the bladder perfusion drugs is reconstituted, the pH of the solution is 4.0 to 5.0 when the concentration of the anthracycline-based chemotherapy drug is 1 to 5 mg/mL. It should be noted that the purpose of defining the reconstitution concentration here is to define the range of the pH value after reconstitution, and is not to limit the reconstitution concentration.

Further, the imidazoquinoline-based immunomodulator includes imiquimod and a derivative thereof, or resiquimod and a derivative thereof, or a soluble salt of imiquimod and the derivative thereof, or a soluble salt of resiquimod and the derivative thereof.

Further, the mass ratio of the anthracycline-based chemotherapy drug to the imidazoquinoline-based immunomodulator is 1:0.1 to 1:10.

Further optionally, the mass ratio of the anthracycline-based chemotherapy drug to imiquimod is 1:1 to 1:10. Further optionally, the mass ratio of the anthracycline-based chemotherapy drug to imiquimod is 1:2 to 1:4.

Further optionally, the mass ratio of the anthracycline-based chemotherapy drug to resiquimod is 1:0.1 to 1:5.

Further, the lyophilized powder formulation further includes a mucosal penetration enhancer.

Further, the mucosal penetration enhancer includes at least one of azone, hyaluronidase, lauryl alcohol, and oleic acid.

Further, the mucosal penetration enhancer can be added in the preparation process or mixed before use.

The compound formulation of the anthracycline-based chemotherapy drug and the immunological adjuvant described in the invention is mixed with the mucosal penetration enhancer before use. The mucosal penetration enhancer can be a medicinal mucosal penetration enhancer available or a mucosal penetration enhancer approved for use in clinical trials.

The invention further provides a method for preparing a lyophilized powder formulation of bladder perfusion drugs.

The method includes the following steps:
S1: dissolving an immunomodulator with a dilute acid to obtain a salt solution of the immunomodulator;
S2: dissolving an anthracycline-based drug with the dilute acid salt solution obtained in S1 to obtain a mixed solution;
and S3: adjusting the pH of the mixed solution to 3.8 to 5.5 with an acidic or alkaline buffer or solution to obtain a final solution, wherein the final concentration of the drug is 1 to 5 mg/mL.

Further, the step S3 further includes:
S31: adding a lyoprotectant into the mixed solution;
further, the step S3 further includes:
   S32: adding a mucosal penetration enhancer into the mixed solution.

Further, the dilute acid in S1 is hydrochloric acid, lactic acid or acetic acid.

Further optionally, the further optional range of pH adjustment in S3 is 4.0 to 5.0.

Further, it further includes step S4: lyophilizing the final solution obtained in S3 to obtain a lyophilized powder formulation.

The invention further provides the application of the lyophilized powder mixed formulation in preparing bladder perfusion drugs. The technical scheme of the invention has the following beneficial effects:
The invention provides a compound formulation of bladder perfusion drugs, which is a mixed lyophilized powder formulation of an anthracycline-based drug and an imidazoquinoline-based immunomodulator. In a large number of experimental analyses, it has been found that the lyophilized powder dosage form is more helpful to protect the long-term chemical stabilities of anthracycline-based drugs in the mixed formulation compared with the traditional injection dosage form. Also, a mixed formulation has a better tumor inhibition effect in the bladder perfusion tumor treatment, can cause a stronger anti-tumor immune response, and compared with the anthracycline-based drug alone, the compound formulation of the invention can reduce the toxic side effects.

The mixed formulation of the anthracycline-based chemotherapy drug and the immunomodulator described in the invention can achieve clinical application through simple reconstitution operation, can be directly perfused for treatment, and can also be perfused after being mixed with a mucosal penetration enhancer to effectively inhibit tumor growth and recurrence. After anthracycline-based drugs cause tumor immunogenic death, immunomodulators can further stimulate antigen-presenting cells to uptake, process and present antigens to T cells, and further amplify the anti-tumor immune response.

The systemic toxic side effects of anthracycline-baseds or immunomodulators can be reduced.

The long-term chemical stability of the mixed formulation of anthracycline-based chemotherapy drugs and immunomodulators is achieved, the generation rate of impurities of anthracycline-based chemotherapy drugs in the presence of immunomodulators is significantly reduced, the long-term storage stability of the formulation is enhanced, and the convenience and safety of the formulation are increased. In the future clinical use, a long shelf life is favorable to the production, storage and transportation of drugs, and is helpful to achieve real clinical application.

Adding a mucosal penetration enhancer into this formulation can increase the retention time of the drug solution on the bladder wall after perfusion treatment and is helpful to further enhance the therapeutic effect of this kind of formulation.

The applicant attempted to directly mix the chemotherapeutic drug epirubicin with imiquimod for perfusion treatment of bladder cancer. The results have shown that, when the ratio of the chemotherapeutic drug epirubicin to imiquimod is in a suitable range, the therapeutic effect can be significantly enhanced compared with that of the chemotherapeutic drug alone, and the toxic and side effects are not increased. It is expected to become a novel formulation of bladder perfusion drugs with significant clinical value. Further study has shown that the anti-tumor mechanism of this novel formulation is mainly that tumor antigens are exposed through inducing the immunogenic cell death of tumor cells by chemotherapeutic drugs such as epirubicin. Also, imiquimod, as a TLR7 agonist, can activate the immune system, recruit antigen-presenting cells into tumors to recognize the tumor antigens, and then activate T cell immune responses against tumors.

However, in the further study of the formulation, we have found that the existence of imiquimod molecules in the simple mixed formulation of a chemotherapeutic drug such as epirubicin and the immunological adjuvant imiquimod will lead to the instability of the molecular structure of the chemotherapeutic drug such as epirubicin. Whether stored in the form of injection or lyophilized powder after mixing, the impurity content in the formulation will increase obviously, and break through the impurity limits of the drugs specified in Pharmacopoeia within a short time. Thus it can't be a stable pharmaceutical formulation which is convenient for clinical application, and the simple mixing of them is not benefit for the chemical stabilities of the drug molecules. Therefore, how to enhance the chemical stability of the compound formulation of epirubicin and imiquimod is the key problem in designing and preparing the compound formulation for using the two in combination.

To solve the above problems, we systematically studied the stability of the compound formulation of epirubicin and imiquimod under various conditions. We have found that epirubicin and imiquimod are more stable under the condition of low pH (such as pH = 3.0) in solution (much better than that under the condition of pH 4.0). However, they will still degrade gradually, resulting in that the formulation cannot be stored for a long time. Thus the injection dosage form is not feasible for this formulation. For lyophilized formulations, we have found that lactose has a significant protective effect on the stability of the formulation, and the increase of lactose content will also be favorable to enhancing the stabilities of the formulations. Nevertheless, the lyophilized formulation prepared under the condition of pH 3.0 cannot be stored for a long time, and it will not pass the inspection of the accelerated stability experiment. Unexpectedly, we have found that this lyophilized compound formulation has a stable "golden pH interval", and the epirubicin/imiquimod compound formulation prepared in this pH range has a very good stability. This phenomenon is contrary to people's conventional understanding of the stability trend of epirubicin single formulation in solution and our investigation results on the stability of the compound formulation in solution.

By further analyzing the impurity spectrum and chemical structure of epirubicin, we have found that the degradation mechanisms of the compound system of epirubicin and imiquimod are different in aqueous solution and lyophilized formulation. First, epirubicin hydrochloride is stable in both injection and lyophilized powder. Currently, epirubicin formulations on the market exist in two dosage forms: injection and lyophilized powder. However, for compound formulations, the existence of imiquimod in solution phase will lead to the rapid rise of an unknown single impurity component of epirubicin. The rate of this reaction will increase with the increase of pH, and it will still occur slowly at the low pH value of pH 3.0 (Note: although the drug solution with lower pH is beneficial to the stability, it will cause great irritation and damage to the bladder when used).

In the solid state from lyophilization, the existence of imiquimod will lead to the rapid rise of Impurity A (the degradation product with sugar ring structure removed) of epirubicin. The reason is speculated that the lone pair electrons of the nitrogen atom on the aromatic ring in the molecular structure of imiquimod attacks the glycosidic bond of epirubicin as a nucleophilic group, leading to the breaking of the chemical bond and the generation of degradation Impurity A. By adding lactose as a lyoprotectant, the direct contact between epirubicin and imiquimod can be blocked to some extent, thus slowing down the occurrence of this reaction. Also, phenomenologically, this reaction is a reaction catalyzed and accelerated by an acid (protonation of oxygen atoms on glycosidic bonds is the intermediate step of the reaction at low pH), thus the increase of pH will significantly slow down the production of Impurity A. On the other hand, we have found that too high pH will lead to the decrease of the solubility of the imiquimod molecule (imiquimod will precipitate from the solution above pH 5.0). Also, too high pH will lead to the increase of another unknown impurity of epirubicin (it is presumed to be the oxidation product of epirubicin). In conclusion, the compound formulation of epirubicin and imiquimod can obtain a dosage form in a suitable pH interval and in the presence of appropriate content of excipients (lactose), which can be stored stably.

In addition, we have also found that adding a mucosal penetration enhancer into this formulation can enhance the penetration and retention of the drug solution in the bladder mucosa after perfusion treatment and is helpful to further enhance the therapeutic effect of this formulation.

The invention provides a mixed lyophilized powder formulation of epirubicin and imiquimod. In a large number of experimental analyses, it has been found that the lyophilized powder dosage form is more helpful to protect the long-term chemical stability of epirubicin in the mixture compared with the traditional injection dosage form. Also, a mixed formulation has a better tumor inhibition effect in the bladder perfusion tumor treatment and can cause a stronger anti-tumor immune response.

The invention provides a compound formulation of epirubicin, including epirubicin or a soluble salt thereof, imiquimod or a soluble salt thereof, a pH regulator and a lyoprotectant.

Further, the compound formulation of epirubicin is a lyophilized powder formulation.

Further, the mass ratio of the epirubicin or the soluble salt thereof to imiquimod or the soluble salt thereof is 1:1 to 1:10.

Further optionally, the mass ratio of epirubicin or the soluble salt thereof to imiquimod or the soluble salt thereof is 1:2 to 1:4.

Further, the pH regulator can be a weak alkali, sodium hydroxide and hydrochloric acid.

Further optionally, the pH regulator is sodium bicarbonate and hydrochloric acid.

Further, the lyoprotectant includes sucrose, lactose, mannitol, and cyclodextrin.

Further, the lyoprotectant is lactose.

Further, the mass fraction of the lyoprotectant in the lyophilized powder formulation is 65% to 96%.

Further, the mass fraction of the lyoprotectant is 80% to 94%.

Further, the mixed lyophilized powder formulation of epirubicin and imiquimod further includes a mucosal penetration enhancer.

Further, the mucosal penetration enhancer includes at least one of azone, hyaluronidase, lauryl alcohol, and oleic acid.

Further, the mucosal penetration enhancer can be added in the preparation process or mixed before use.

Further, the pH of the mixed formulation of epirubicin and imiquimod before lyophilization is 3.8 to 5.5. It should be noted that the purpose of defining the reconstitution concentration here is to define the range of the pH value after reconstitution, and is not to limit the reconstitution concentration.

Further, the pH of the mixed lyophilized powder formulation of epirubicin and imiquimod after reconstitution is 3.8 to 5.5 when the concentration of epirubicin is 1 to 5 mg/mL. It should be noted that the purpose of defining the reconstitution concentration here is to define the range of the pH value after reconstitution, and is not to limit the reconstitution concentration.

Further, the pH of the lyophilized powder dosage form after reconstitution is 4.0 to 5.0 when the concentration of epirubicin is 1 mg/mL.

Further optionally, the pH values of the lyophilized powder dosage forms after reconstitution are 4.0 to 4.4, 4.4 to 5.0 and 5.0 to 5.5 when the concentration of epirubicin is 1 mg/mL.

The invention provides a method for preparing a mixed lyophilized powder formulation of epirubicin and imiquimod.

The method includes the following steps:
S1, dissolving imiquimod or the soluble salt thereof with a dilute acid to obtain a dilute acid salt solution of imiquimod;
S2: dissolving epirubicin with the solution in S1 to obtain a mixed solution;
and S3: adjusting the pH of the mixed solution to 3.8 to 5.5 with an alkaline buffer or alkaline solution;
further, the step S3 further includes:
   S31: adding a mucosal penetration enhancer into the mixed solution;

Further, the dilute acid solution in S1 is hydrochloric acid, lactic acid and acetic acid.

Further, the preferred range of pH adjusted in S3 is 4.0 to 5.0.

Further optionally, the pH values adjusted in S3 can be 3.8 to 4.0, 4.0 to 4.4 and 4.4 to 5.0.

Further, it further includes S4: lyophilizing the final solution obtained in S3 to obtain a lyophilized powder formulation.

The invention further provides the application of the lyophilized powder mixed formulation in preparing bladder perfusion drugs.

The technical scheme of the invention has the following beneficial effects:
The mixed formulation of epirubicin and imiquimod described in the invention can achieve clinical application through simple reconstitution operation, and can effectively inhibit tumor growth and recurrence through the method of perfusion. After epirubicin causes tumor immunogenic death, imiquimod can further stimulate antigen-presenting cells to uptake, process and present antigens to T cells, and further amplify the anti-tumor immune response.

The long-term chemical stability of the mixed formulation of epirubicin and imiquimod is achieved, the generation rate of impurities of epirubicin in the presence of imiquimod is significantly reduced, the long-term storage stability of the formulation is enhanced, and the convenience and safety of the formulation are increased.

In the present application, the terms "immunological adjuvant" and "immunomodulator" are generally used interchangeably. In certain embodiments, the immunological adjuvant itself can exist in the form of a soluble salt, or it can be converted into the form of a soluble salt by certain operations, so the term "immunological adjuvant" includes an immunological adjuvant or a soluble salt thereof.

In the present application, the term "lyoprotectant" can be used interchangeably with "lyophilization cosolvent".

In the present application, the term "pharmaceutical composition" can generally refer to a composition including a pharmaceutically active ingredient. The pharmaceutical composition can also contain other ingredients. For example, the pharmaceutical composition can also include a pharmaceutically acceptable carrier, for example, a pH regulator, for example, a lyoprotectant. In the present application, the term "pharmaceutical composition" also encompasses the case of compound formulations.

For example, the bladder perfusion pharmaceutical composition can be a compound formulation of bladder perfusion drugs, for example, the compound formulation of the bladder perfusion drugs can include anthracycline-based chemotherapy drugs and immunological adjuvants (immunomodulators). For example, the compound formulation of the bladder perfusion drugs can further include a pH regulator.

For example, the bladder perfusion pharmaceutical composition can be a compound formulation of epirubicin. For example, the compound formulation of epirubicin can include epirubicin or a soluble salt thereof, imiquimod or a soluble salt thereof, and a pH regulator.

Not to be limited by any theory, the following examples are only used to illustrate the bladder perfusion pharmaceutical composition, preparation method and use of this application, etc., and are not intended to limit the scope of the invention of this application.

### EXAMPLES

The names, specific structures and reference substance CAS of the impurities involved in the Examples are as follows:

| Name | Structure | Chemical name | Reference CAS of impurity |
|---|---|---|---|
| Impurity A of imiquimod | 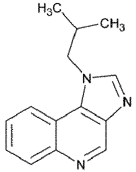 | 1-isobutyl-1H-imidazo[4,5-C]quinoline | 99010-24-9 |
| Impurity B of imiquimod | 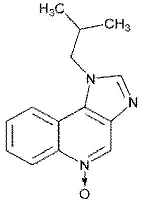 | 1-isobutyl-1H-imidazo[4,5-C]quinoline-N-oxide | 99010-63-6 |
| Impurity C of imiquimod | 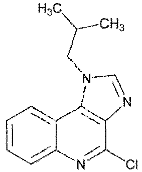 | 1-(2-methylpropyl)-4-chloro-1H-imidazo[4,5-c]quinoline | 99010-64-7 |
| Impurity A of epirubicin hydrochloride (EPI-A) | 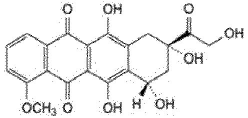 | doxorubicinone | 24385-10-2 |
| Impurity C of epirubicin hydrochloride (EPI-C) | 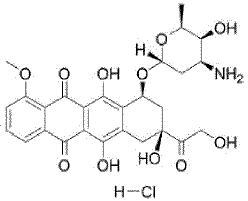 | doxorubicin hydrochloride | 23214-92-8 |

### Example A Preparation and combination

Example A1: 10 mg R837 was weighed, into which was added 5 mL of 0.015 M hydrochloric acid, and shaken until the solution was completely clear. The ICD chemotherapeutic drug doxorubicin hydrochloride was added into the solution, with the mass ratio of imiquimod to doxorubicin hydrochloride of 1:1, 2:1, 4:1, 6:1 and 10:1. A suitable lyophilization cosolvent such as mannitol was added, and a pH regulator such as sodium hydroxide was added to control the pH value between 2.0 and 5.5, then the mixture was lyophilized into a lyophilized powder.

Example A2: 10 mg R837 was weighed, into which was added 5 mL of 0.015 M hydrochloric acid, and shaken until the solution was completely clear. The ICD chemotherapeutic drug epirubicin hydrochloride was added into the solution, with the mass ratio of imiquimod to epirubicin hydrochloride of 1:1, 2:1, 4:1, 6:1 and 10:1. A suitable lyophilization cosolvent such as mannitol was added, and a pH regulator such as sodium hydroxide was added to control the pH value between 2.0 and 5.5, then the mixture was lyophilized into a lyophilized powder.

Example A3: 10 mg R837 was weighed, into which was added 5 mL of 0.015 M hydrochloric acid, and shaken until the solution was completely clear. The ICD chemotherapeutic drug pirarubicin hydrochloride was added into the solution, with the mass ratio of imiquimod to pirarubicin hydrochloride of 1:1, 2:1, 4:1, 6:1 and 10:1. A suitable lyophilization cosolvent such as mannitol was added, and a pH regulator such as sodium hydroxide was added to control the pH value between 2.0 and 5.5, then the mixture was lyophilized into a lyophilized powder.

Example A4: 10 mg R837 was weighed, into which was added 5 mL of 0.015 M hydrochloric acid, and shaken until the solution was completely clear. The ICD chemotherapeutic drug mitoxantrone hydrochloride was added into the solution, with the mass ratio of imiquimod to mitoxantrone hydrochloride of 1:1, 2:1, 4:1, 6:1 and 10:1. A suitable lyophilization cosolvent such as mannitol was added, and a pH regulator such as sodium hydroxide was added to control the pH value between 2.0 and 5.5, then the mixture was lyophilized into a lyophilized powder.

Example A5: 10 mg R837 was weighed, into which was added 5 mL of 0.015 M lactic acid or acetic acid (hydrochloric acid was not allowed), and shaken until the solution was completely clear. The ICD chemotherapeutic drug oxaliplatin was added into the solution, with the mass ratio of imiquimod to oxaliplatin of 1:1, 2:1, 4:1, 6:1 and 10:1. A suitable lyophilization cosolvent such as mannitol was added, and a pH regulator such as sodium hydroxide was added to control the pH value between 4.5 and 6.0, then the mixture was lyophilized into a lyophilized powder.

Example A6: 2 mg R837 was weighed, into which was added 5 mL of 0.005 M hydrochloric acid, and shaken until the solution was completely clear. The ICD chemotherapeutic drug fluorouracil was added into the solution, with the mass ratio of imiquimod to fluorouracil of 1:1, 1:2, 1:5, 1:10 and 1:20. A suitable lyophilization cosolvent such as mannitol was added, and a pH regulator such as sodium hydroxide was added to control the pH value between 3.0 and 6.0, then the mixture was lyophilized into a lyophilized powder.

Example A7: 2 mg R848 was weighed, into which was added 5 mL of 0.005 M hydrochloric acid, and shaken until the solution was completely clear. The ICD chemotherapeutic drug doxorubicin hydrochloride was added into the solution, with the mass ratio of R848 to doxorubicin hydrochloride of 1:1, 1:2, 1:5, 1:10 and 1:20. A suitable lyophilization cosolvent such as mannitol was added, and a pH regulator such as sodium hydroxide was added to control the pH value between 2.0 and 5.5, then the mixture was lyophilized into a lyophilized powder.

Example A8: 2 mg R848 was weighed, into which was added 5 mL of 0.005 M hydrochloric acid, and shaken until the solution was completely clear. The ICD chemotherapeutic drug epirubicin hydrochloride was added into the solution, with the mass ratio of R848 to epirubicin hydrochloride of 1:1, 1:2, 1:5, 1:10 and 1:20. A suitable lyophilization cosolvent such as mannitol was added, and a pH regulator such as sodium hydroxide was added to control the pH value between 2.0 and 5.5, then the mixture was lyophilized into a lyophilized powder.

Example A9: 1 mg R848 was weighed, into which was added 2.5 mL of 0.005 M hydrochloric acid and then 5-15 mL of water for injection, and shaken until the solution was completely clear. The ICD chemotherapeutic drug fluorouracil was added into the solution, with the mass ratio of R848 to fluorouracil of 1:10, 1:25, 1:100 and 1:200. A suitable lyophilization cosolvent such as mannitol was added, and a pH regulator such as sodium hydroxide was added to control the pH value between 3.0 and 6.0, then the mixture was lyophilized into a lyophilized powder.

Example A10: Gemcitabine hydrochloride and CpG were mixed in aqueous solution according to the mass ratio of 100:1, into which were added the suitable lyophilization cosolvents mannitol and lactose, and the pH value was controlled between 2.5 and 4.0, and then the mixture was lyophilized into a lyophilized powder.

### Example B Design of comparative experiments and implementation of application

Example B1: The combined drug system was used in the perfusion treatment of bladder cancer, and the therapeutic effect was significantly increased compared with the chemotherapeutic drug alone or the immunological adjuvant alone (imiquimod).

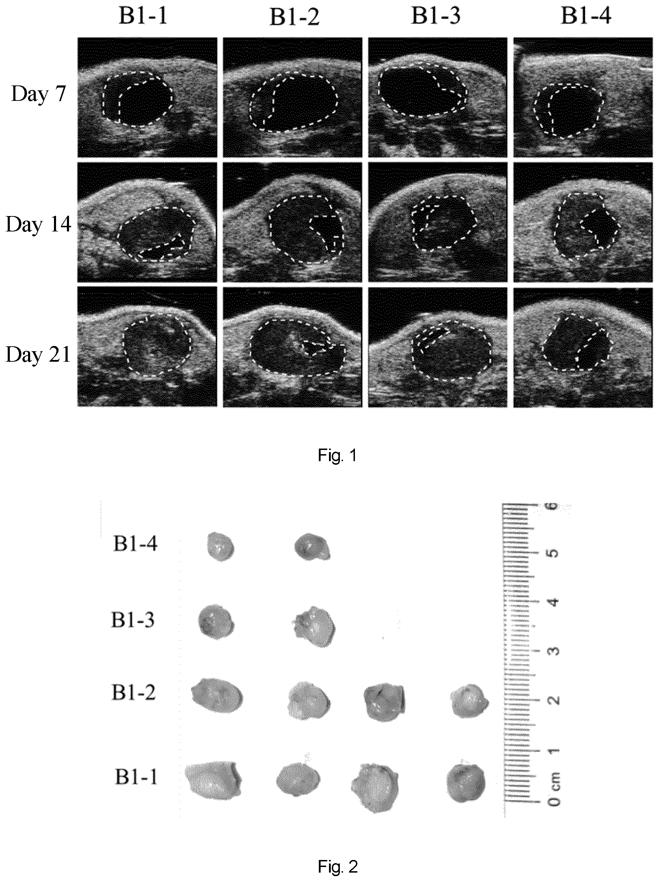

Incorporating with Fig. 1, the effect of combined infusion of doxorubicin hydrochloride (which was called DOX hereinafter for short) and imiquimod hydrochloride (which was called R837 hereinafter for short) in the treatment of bladder in situ tumor was evaluated. Healthy C57BL/6 female mice (18-20 g) were divided into four groups, with 4 mice in each group. In situ bladder cancer models were established by injecting fLuc-MB49 cells (1×10⁵ cells) into the bladder walls. One week after inoculation, the mice were imaged in vivo to confirm the establishment of the tumor model, and then the mice were anesthetized by intraperitoneal injection of pentobarbital, and the following drug combinations were perfused into the bladders respectively:
Group B1-1: blank group (ultrapure water ddH₂O, 100 µL);
Group B1-2: R837 (1.5 mg/mL, 100 µL);
Group B1-3: DOX (1.5 mg/mL, 100 µL);
And Group B1-4: DOX+R837 (1.5 mg/mL, 100 µL, 1:1)_{∘}

The perfusion time was 1 hour, once a week, twice in total. The sizes of the *in situ* tumor were detected by a photoacoustic imaging system before treatment every week. Fig. 1 is a photoacoustic image of bladder in situ tumor, in which the area inside the outer dotted line is the mouse bladder area and the middle area between the two dotted lines is the tumor tissue. By comparing the area of the regions between the two dotted lines, it can be determined that the inhibitory effect of the combined perfusion group of R837 and DOX (B1-4) on bladder in situ tumor is significantly stronger than that of the group of R837 alone (B1-2) and the group of DOX alone (B1-3); Fig. 2 is an image of the tumors of bladder in situ tumor obtained by dissecting mice one week after the second perfusion treatment. The results have shown that in the group using DOX and R837 in combination to perfuse (B1-4), the volumes of bladder in situ tumor in the mice are smaller, and the tumor inhibition effect is more significant. Therefore, it is concluded that using DOX and R837 in combination to perfuse can enhance the therapeutic effect of the chemotherapeutic drug.

Example B2: Evaluation of the experiment using R837 hydrochloride and pirarubicin hydrochloride (which was called THP hereinafter for short) in combination for perfusion treatment of bladder in situ tumor. The drugs mentioned above are hydrochloride of the chemotherapeutic drug and hydrochloride of R837. The clinical dosage form of R837 is ointment, which is not suitable for perfusion application. Currently, the patent dosage form of R837 for bladder perfusion is emulsion (TMX-1 01). Here, we investigated the therapeutic effect of using R837 hydrochloride and THP in combination.

Bladder in situ tumor of mice was established by a similar method as described in Example B1. One week after inoculation, the mice were imaged in vivo to confirm the establishment of the tumor model, and randomly divided into three groups. Then the mice were anesthetized by intraperitoneal injection of pentobarbital, and the following drug combinations were perfused into the bladders respectively:
Group B2-1: blank group (ultrapure water ddH₂O, 100 µL);
Group B2-2: THP (0.6 mg/mL, 100 µL);
Group B2-3: THP (0.6 mg/mL, 100 µL) + R837 (0.6 mg/mL, 100 µL).

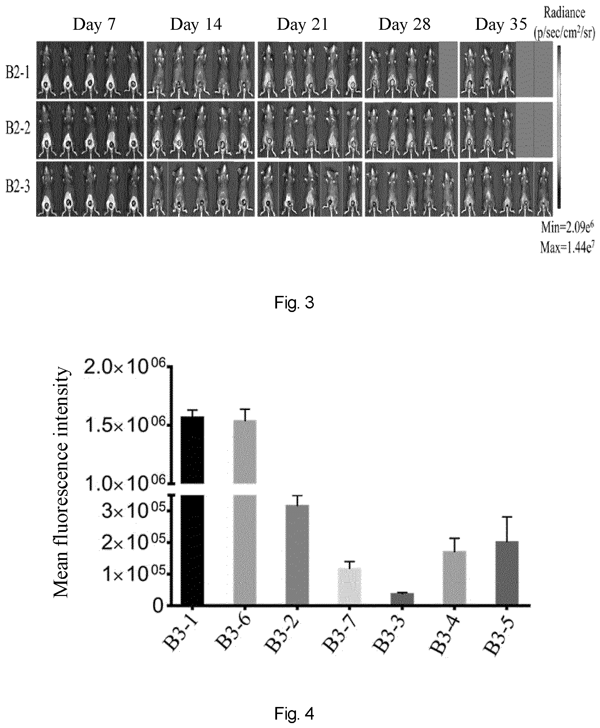

Bladder perfusion was carried out for 1 hour, once a week, four times in total. Before each perfusion treatment, bladder tumor in mice was detected by employing a small animal imaging system, and the tumor sizes were characterized by the mean fluorescence intensities of the tumor tissues. The in-vivo imaging data of the mice from 0 to 35 days are shown in Fig. 3. The mean fluorescence intensities of the bladder in situ tumor tissues of the mice in Fig. 3 and the survival rates of each group are shown in Table 1. The fluorescence intensities of the tumors of the bladder sites of the mice in the combined perfusion group of THP and R837 (B2-3) are significantly lower than that of other control groups, and the mice in this group have good survival rates (100%) until 35 days. Therefore, it is concluded that because hydrochloride of R837 has good water solubility and high perfusion bioavailability, using hydrochloride of R837 and THP in combination can improve the tumor inhibition effect of the chemotherapeutic drug and increase the survival rate of tumor-bearing mice.

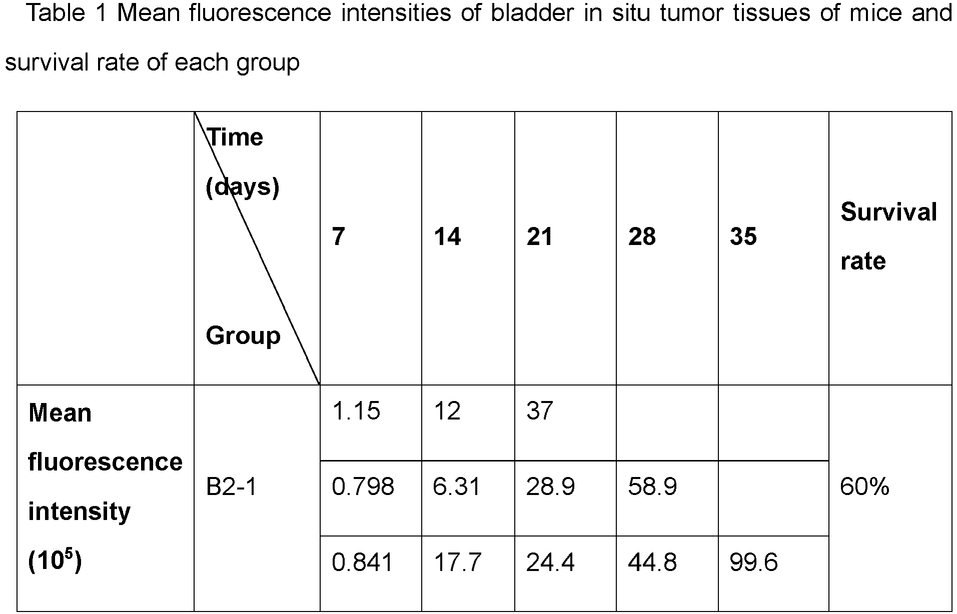

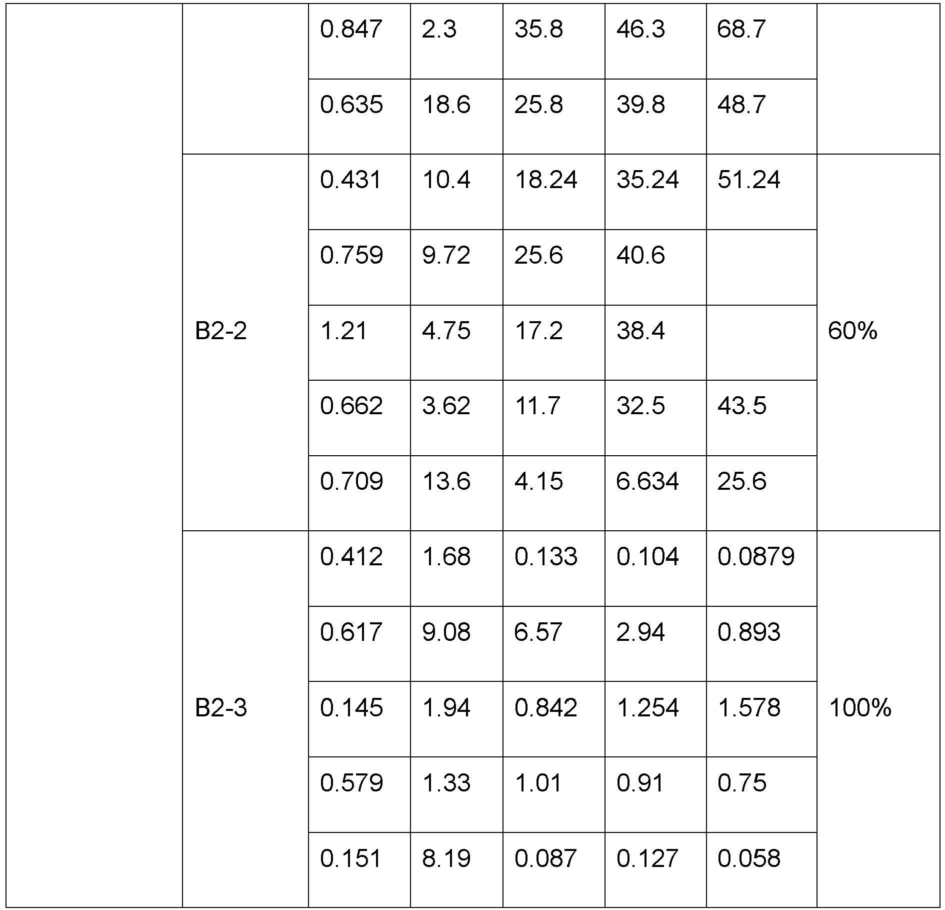

Example B3: Evaluation of the effect of the combined perfusion of pirarubicin hydrochloride (THP for short hereinafter) and R837 hydrochloride in different ratios for the treatment of bladder in situ tumor.

Bladder in situ tumor of mice was established by a similar method as described in Example B1. To simulate the perfusion treatment after resection of bladder tumor in clinic, this experiment adopted the treatment scheme of perfusion treatment on the second day after inoculation. Before treatment, the tumor growth was detected by a small animal in vivo imaging system, and the tumor inhibition levels were evaluated by the mean fluorescence intensities of the tumor tissues. The combination of bladder perfusion drugs in the experiment is as follows:
Group B3-1: blank group (ultrapure water ddH₂O, 100 µL);
Group B3-2: THP (0.25 mg/mL, 100 µL);
Group B3-3: THP (0.25 mg/mL, 100 µL) + R837 (0.8 mg/mL, 100 µL);
Group B3-4: THP (0.25 mg/mL, 100 µL) + R837 (0.4 mg/mL, 100 µL);
Group B3-5: THP (0.25 mg/mL, 100 µL) + R837 (0.2 mg/mL, 100 µL);
Group B3-6: R837 (0.8 mg/mL, 100 µL);
Group B3-7: THP (0.25 mg/mL, 100 µL) + BCG (2.6 mg/mL, 100 µL).

Perfusion was carried out for 1 hour every week, three times in total. On day 16, the fluorescence statistical data of bladder in situ tumor of the mice in each group are shown in Fig. 3, in which the higher the mean fluorescence intensity, the larger the tumor tissue. In Fig. 3, the mean fluorescence intensities of the group of R837 alone (B3-6) and that of the blank group (B3-1) have no significant difference, indicating that R837 alone has no significant tumor inhibition effect at this dose. The tumor fluorescence intensities of the group using THP and R837 in combination (B3-3 to 5) are significantly lower than that of the group of THP alone and BCG positive control group (B3-7), indicating that using THP and R837 in combination has a significant synergistic therapeutic effect, the therapeutic effect will be enhanced with the increase of the R837 ratio, and the therapeutic effect is obviously better than that of BCG positive control group. Therefore, the above data once again verify the conclusion that using R837 and ICD drugs in combination has synergistic anti-tumor effect.

Example B4: Evaluation of the experiment using epirubicin hydrochloride (EPI for short hereinafter) and hydrochloride of R837 in different ratios in combination for perfusion treatment of bladder in situ tumor.

To further verify the conclusion that combined perfusion of ICD drugs and TLR7 agonists can enhance the therapeutic effect of chemotherapeutic drugs and reduce the toxic and side effects of chemotherapeutic drugs, we evaluated the therapeutic effect of combined perfusion of EPI (60 µg) and hydrochloride of R837 (60-240 µg) in different ratios on bladder in situ tumor in mice. Taking the non-ICD drug mitomycin C (MMC) as the control, the results are shown in Fig. 4. The in situ tumor model in mice were established by the method in Example B1. Perfusion treatment was started 5 days after the mice were inoculated with cells (which was set as day 0). The mice were randomly divided into the following groups:
Group B4-1: blank group (ultrapure water ddH₂O);
Group B4-2: R837 (240 µg);
Group B4-3: EPI (60 µg);
Group B4-4: EPI (60 µg) + R837 (60 µg);
Group B4-5: EPI (60 µg) + R837 (120 µg);
Group B4-6: EPI (60 µg) + R837 (240 µg);
Group B4-7: MMC (60 µg) + R837 (120 µg).

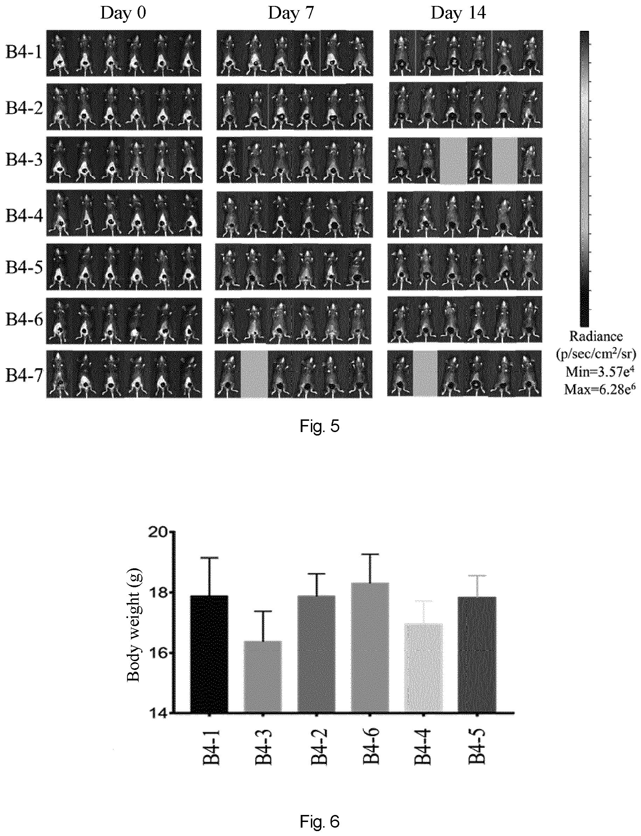

Each group of the mice was perfused with a volume of 100 µL each time for 1 hour, once a week, twice in total. Fig. 5 shows the small animal fluorescence in vivo imaging before the first perfusion treatment and one week after the second perfusion treatment in the mice. Table 2 shows the mean fluorescence intensities of bladder in situ tumor of the mice and the survival rates of the mice in each group on the 14th day. According to the results of this experiment, with the increase of the administration ratio of hydrochloride of R837 used in combination with EPI, the mean fluorescence intensity of in situ tumor of the mice is weakened, and the survival time of the mice is improved. Mice in the group using MMC and R837 in combination die in the early stage of the experiment due to MMC toxicity, thus the combined application effect of EPI and R837 is obviously better than that of MMC and R837 in combination. Fig. 6 shows the body weights of the mice in each group on day 9 after administration. Comparing the data in the bar chart, the body weights of the mice in the group of EPI alone (B4-2) are significantly lower than that in the blank group (B4-1), while the losses of the body weights of the mice in the group using R837 in combination (B4-4 to 6) is decreased with the increase of the R837 ratio, thus suggesting the attenuation effect of using ICD drugs and R837 in combination.

**Table 2 Mean fluorescence intensities of bladder in situ tumor tissues of mice and survival rates of each group**

| **Group** | | **B4-1** | **B4-2** | **B4-3** | **B4-4** | **B4-5** | **B4-6** | **B4-7** |
|---|---|---|---|---|---|---|---|---|
| **Mean fluorescence intensity (10⁵)** | Day 0 | 1.87 | 1.8 | 2.09 | 2.36 | 2.15 | 2.58 | 1.98 |
| | | 2.2 | 1.69 | 1.62 | 2.05 | 1.07 | 1.75 | 2.25 |
| | | 2.17 | 1.03 | 1.84 | 1.07 | 2.24 | 1.34 | 2.21 |
| | | 1.74 | 2.7 | 2.24 | 1.64 | 1.21 | 1.08 | 1.95 |
| | | 2.15 | 3.04 | 1.22 | 0.11 | 0.98 | 0.9 | 0.95 |
| | | 0.9 | 1.0 | 2.73 | 0.99 | 1.09 | 1.25 | 1.42 |
| | Mean | 1.838 | 1.877 | 1.957 | 1.37 | 1.457 | 1.483 | 1.793 |
| | Standard deviation | 0.457 | 0.770 | 0.478 | 0.746 | 0.527 | 0.556 | 0.464 |
| | Day 7 | 5.67 | 6.37 | 2.89 | 1.53 | 0.91 | 1.43 | 1.67 |
| | | 7.57 | 5.49 | 2.36 | 1.51 | 1.3 | 0.15 | |
| | | 5.2 | 5.35 | 4.7 | 2.67 | 2.68 | 0.51 | 0.52 |
| | | 5.1 | 5.35 | 4.72 | 0.95 | 0.295 | 0.36 | 1.94 |
| | | 5.2 | 6.92 | 5.62 | 0.26 | 0.39 | 0.59 | 3.24 |
| | | 6.2 | 6.05 | 3.07 | 2.7 | 0.43 | 0.52 | 3.21 |
| | Mean | 5.823 | 5.922 | 3.893 | 1.603 | 1.001 | 0.593 | 2.116 |
| | Standard deviation | 0.868 | 0.585 | 1.180 | 0.874 | 0.828 | 0.401 | 1.023 |
| | Day 14 | 13.8 | 7.97 | 12.8 | 0.89 | 1.12 | 0.81 | 8.22 |
| | | 10.7 | 10.2 | 10.9 | 2.0 | 0.47 | 0.21 | |
| | | 10.3 | 9.04 | | 1.7 | 0.4 | 1.04 | 1.08 |
| | | 13.8 | 12.7 | | 1.7 | 4.69 | 1.02 | 7.09 |
| | | 15.5 | 7.53 | | 1.64 | 0.96 | 1.43 | 10.9 |
| | | 11.0 | 8.28 | 8.7 | 10.5 | 2.49 | 5.14 | 8.04 |
| | Mean | 12.517 | 9.287 | 10.8 | 3.072 | 1.688 | 1.608 | 7.066 |
| | Standard deviation | 1.945 | 1.750 | 1.675 | 3.339 | 1.508 | 1.621 | 3.250 |
| **Survival rate** | | 100% | 100% | 67% | 100% | 100% | 100% | 83% |

### Example C Evaluation of the effects of bladder perfusion pharmaceutical compositions

Example C1: Evaluation experiment of using ICD drugs and R837 in combination for the perfusion treatment of bladder cancer and the inhibition of distal tumor growth.

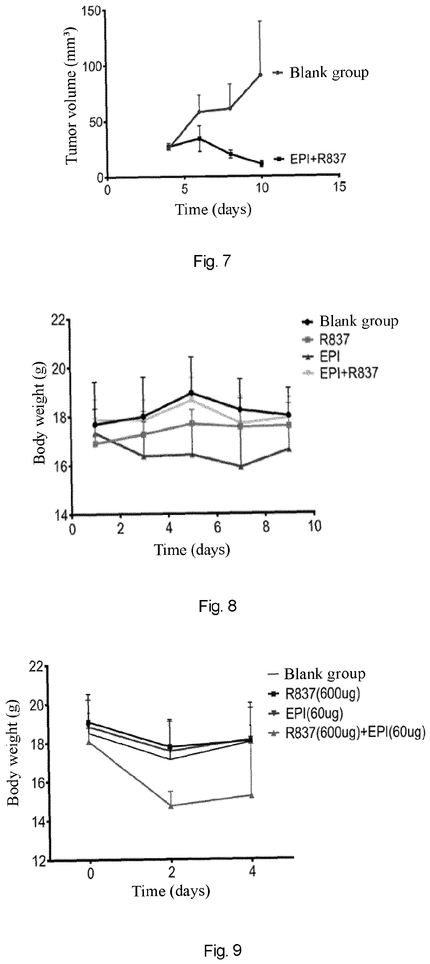

The above experiments confirm the inhibitory effect of using the ICD drug EPI and the immunological adjuvant R837 in combination on the growth of bladder in situ tumor and the attenuation effect on chemotherapy, and the combined therapeutic effect is more significant with the increase of the dose of the immunological adjuvant R837 in a certain dose range. On day 14, the EPI+R837 (360 µg) group showed a very significant therapeutic effect on tumor in-situ in the mice. To further evaluate the inhibitory effect of the specific immune function produced by using the ICD drug and R837 in combination on the growth of distant tumors, two weeks after the completion of the perfusion treatment (the third time) on day 14 (day 28), the mice in EPI+R837 (360 µg) group and mice in healthy blank group (C57 BL/6) were inoculated subcutaneously with MB49 cells (1×10⁶ cells), and the tumor growth was observed. From day 4 after subcutaneous tumor inoculation, the sizes of subcutaneous tumors were measured and recorded every other day. The formula for calculating the tumor volume was V= (length× width²)/2. The data of subcutaneous tumor volume of the mice in Fig. 7 can show that the subcutaneous tumor volumes of the mice in EPI+R837 group are obviously smaller than that in the blank control group, which proves that using EPI and R837 in combination has produced a tumor-specific immune and distant tumor inhibition effect, which can reduce the recurrence and metastasis of bladder tumor.

Example C2: Evaluation of the attenuation effect and mechanism of using ICD drugs and R837 in different mass ratios (1:6, 1:10).

To further confirm the conclusion that using R837 and ICD drugs in combination can weaken the toxic side effects of chemotherapeutic drugs and the maximum dose range of using R837 and ICD in combination, we selected healthy C57BL/6 female mice for further toxicity evaluation. Six mice in each of the four groups were perfused with the same dose of blank ultrapure water (ddH2O), R837 (360 µg), EPI (60 µg) and EPI (60 µg) + R837 (360 µg) for 1 hour, once a week, twice, and the body weight was recorded every other day. As shown in Fig. 8, after two times of perfusion administration (1-9 days), the body weights of the mice in the EPI (60 µg) group are significantly lower than that in the blank group and R837 (360 µg) group, but the body weights of the mice in the group using the same administration dosage of EPI and R837 (1: 6) in combination are not significantly lower, and the effect will be proved by the experiment of phagocytosis of macrophages. Next, we used a similar method to evaluate whether there was a usage range for the attenuation effect when EPI and R837 were used in combination in a mass ratio (1:10). Six mice in each of the four groups were perfused with the same dose of blank ultrapure water (ddH₂O), R837 (600 µg), EPI (60 µg) and EPI (60 µg) + R837 (600 µg) for 1 hour, and the body weights was recorded every other day. As shown in Fig. 9, the body weights of the mice in the group of using EPI and R837 (1:10) in combination decrease obviously compared with other control groups. It is speculated that when the mass ratio of R837 continues to increase, the increase of R837 dosage leads to the increase of new toxic side effects, which counteract the attenuation effect that would otherwise occur when using in combination. Also, incorporating with the experimental results in Example Part B, it can be concluded that the combined perfusion administration of R837 and ICD chemotherapeutic drugs in a proper combination dose range can significantly reduce the toxic side effects of the chemotherapeutic drugs.

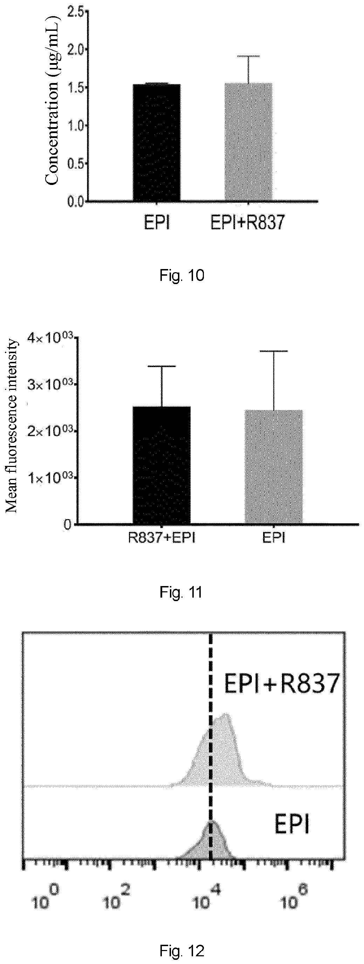

To explain why using in combination can attenuate, the difference of the content of the chemotherapeutic drug in blood and bladder tissues of the mice 4 hours after bladder perfusion administration was investigated. Fig. 10 shows the contents of EPI in the blood of the mice after 4 hours of perfusion administration detected by a fluorescence spectrophotometer. The experimental data show that there is no significant difference in the concentrations of the chemotherapy drug EPI in the blood of the mice in the EPI group and the group of using EPI+R837 in combination. Also, the total phagocytoses of EPI in bladder tissues was evaluated by flow analysis. Fig. 11 shows the data of EPI flow fluorescence analysis of the bladder tissue cells of the mice. The experimental results have shown that there is no significant difference in the uptake rates in the bladder tissues of the mice between using EPI alone and using EPI and R837 in combination, that is, the using in combination with R837 will not affect the systemic and local absorption of the chemotherapeutic drug EPI. However, it has been found in the experiment of the flow analysis detection of the EPI positive proportions of macrophages in the bladder tissues of mice that, the abilities of the macrophages in the bladder tissues in the group using EPI and R837 in combination to phagocytose chemotherapeutic drugs are stronger than that in the group of EPI alone (Fig. 12). Incorporating with the above-mentioned phenomenon of the improvement of the therapeutic effect, it can be concluded that after the immunogenic cell death caused by chemotherapeutic drugs, macrophages will phagocytose a large number of extracellular free chemotherapeutic drugs, thus reducing the toxic side effects of the extracellular chemotherapeutic drugs on normal tissues. However, R837, as an immunostimulant, has the effect of immune regulation. Although increasing the ratio of R837 in a proper amount will reduce the toxic and side effects of chemotherapy drugs, using excessive R837 in combination with chemotherapy drugs will cause strong mucosal irritation and have adverse effects on mice. Therefore, through this example, it is proved that the preferred chemotherapy drug/R837 ratio is not less than 1:10. Therefore, when immunological adjuvants and chemotherapy drug capable of causing immunogenic cell death are used in combination as a bladder perfusion pharmaceutical composition, there is competition between attenuation and new toxic and side effects. Thus there is a certain ratio relationship in a pharmaceutical composition. Although the differences in the nature of different drugs lead to differences in the ratio relationship, when immunological adjuvants and chemotherapy drug capable of causing immunogenic cell death are used in combination as a bladder perfusion pharmaceutical composition, the systemic toxic side effects of ICD chemotherapy drugs can be reduced through the technical scheme claimed in the invention, the usage amount of ICD drugs can also be enhanced in a certain range, and the therapeutic effect of the drugs is obviously enhanced.

### Example D Study on the stabilities of injection dosage forms

The compound formulation of epirubicin and R837 was prepared without lyophilization to investigate the changes in the impurities in the injection dosage forms under different pH conditions and temperature conditions, and to determine whether the injection forms meet the actual production and use of the compound formulation.

Example D1: Chemical stability of the injection formulation of the bladder perfusion pharmaceutical composition under different pH conditions of drug solutions.

Imiquimod was dissolved with hydrochloric acid, and then epirubicin hydrochloride was added for dissolution. The pH value of the solution was low, which would cause great irritation to the tissues during perfusion. To reduce the irritation to the tissues during use, it was necessary to choose a pH value that takes into account both the stability of the formulation and the use safety.

R837 was dissolved with hydrochloric acid to obtain a hydrochloride solution of R837 with a concentration of 4 mg/mL, and then epirubicin hydrochloride (EPI) was added for dissolution with a concentration of EPI of 1 mg/mL. Then sodium bicarbonate was used to adjust the pH of the solutions to 3.0, 3.5 and 4.0, which were labeled as Injection 1, Injection 2 and Injection 3 respectively. The contents of impurities of epirubicin and imiquimod in the pharmaceutical formulations on the day of preparation and day 5 after being placed in an environment of 40°C were detected by liquid chromatography, and the results are shown in Table 3. The impurities mainly come from epirubicin hydrochloride, and the structure of Impurity A (EPI-A) is 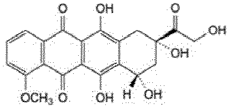 and that of Impurity C (EPI-C) is

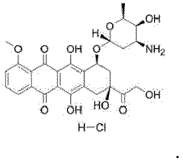

The results have shown that under the three pH conditions, the total impurity content of the formulation increases with time compared with that of the bulk drugs, and the total impurity content is more than three times that of the bulk drugs on day 5 after being placed in an environment of 40°C. Even under the most stable condition of pH=3.0, an unknown single impurity of EPI increases significantly after only 5 days of storage at 40°C, which is expected to exceed the maximum allowable limits specified in the Pharmacopoeia soon. Also, it has been found that the impurity content of imiquimod is relatively stable. In many tests, Impurity B of imiquimod was kept at about 0.05%, Impurity A of imiquimod was kept at about 0.05%, and Impurity C of imiquimod was not detected. The maximum unknown single impurity was kept at about 0.18%, and the total impurities was substantially within 0.39%, none of which exceeded the limit: Impurity A content 0.15%, Impurity B content 0.20%, Impurity C content 0.15%, the maximum unknown single impurity content 0.20%, and total impurity content 1.0%. Therefore, the compound injection dosage form of epirubicin/imiquimod does not have good stability, and the main impurity growth comes from epirubicin, thus the safety and effectiveness of clinical medication cannot be guaranteed.

**Table 3 The record table of the initial impurity contents of epirubicin in the compound injection dosage forms of epirubicin/imiquimod at different pH values and the impurity contents of epirubicin (EPI) after standing at 40°C for 5 days .**

| Sample number and pH | Time (days ) | EPI-A (%) | EPI-C (%) | Unknown single impurity (%) | Total impurity (%) |
|---|---|---|---|---|---|
| EPI (bulk drug)-Day 0 | | 0.18 | 0.21 | 0.09 | 0.71 |
| Injection 1, pH3.0 | 0 | 0.15 | 0.23 | 0.12 | 0.79 |
| | 5 | 0.41 | 0.22 | 0.32 | 2.06 |
| Injection 2, pH3.5 | 0 | 0.15 | 0.24 | 0.19 | 0.85 |
| | 5 | 0.26 | 0.20 | 0.56 | 2.49 |
| Injection 3, pH4.0 | 0 | 0.15 | 0.21 | 0.28 | 1.06 |
| | 5 | 0.21 | 0.19 | 0.90 | 3.31 |
| **Limit** | | **1.0** | **1.0** | **0.5** | **2.5** |

### Example D2: Effect of different storage conditions on the stabilities of injection dosage forms

Considering that the storage temperature of the formulation in Example D1 was 40°C, it might accelerate the generation of impurities, thus the stability of the formulation at lower temperature was explored. Bladder perfusion injections with pH=3.0 and pH=3.5 were prepared by the method in Example D1, which were placed under a cold storage condition, at 15°C and at 25°C. The impurity contents in the formulation samples were detected on the day of the sample formulation and day 10 after being placed at different temperatures. The results are shown in Table 4. With the increase of storage temperature, the total impurity contents of the injections under two pH conditions all increase, which confirms that the stability of the injection dosage form is related to temperature. Also, it has been found that even under the cold storage condition, the maximum unknown impurity is still increasing, and the increase of the maximum unknown impurity is also not benefit for the overall stability and safety of the formulation. Therefore, the injection dosage form for bladder perfusion is probably not the preferred dosage form of the formulation.

**Table 4 The record table of the initial impurity contents of epirubicin (EPI) in the compound injection formulations of epirubicin/imiquimod and the impurity contents of epirubicin (EPI) in the compound injection formulation of epirubicin/imiquimod on day 10 after standing at different storage temperatures.**

| pH | Time (days) | Temperature (°C) | EPI-A | EPI-C | Maximum unknown impurity (%) | Total impurity (%) |
|---|---|---|---|---|---|---|
| 3.0 | 0 | - | 0.23 | 0.31 | 0.13 | 0.8 |
| | 10 | Cold storage | 0.16 | 0.26 | 0.17 | 0.75 |
| | | 15 | 0.17 | 0.22 | 0.24 | 1.1 |
| | | 25 | 0.24 | 0.24 | 0.47 | 1.6 |
| 3.5 | 0 | - | 0.22 | 0.31 | 0.13 | 0.82 |
| | 10 | Cold storage | 0.13 | 0.23 | 0.28 | 0.93 |
| | | 15 | 0.12 | 0.24 | 0.47 | 1.4 |
| | | 25 | 0.19 | 0.26 | 0.59 | 1.9 |
| **Limit** | | | **1.0** | **1.0** | **0.5** | **2.5** |

### Example E Investigation on the stabilities of lyophilized dosage forms

The results in Example D show that the compound formulation is difficult to be produced, stored and used as an injection, thus an experiment was designed to investigate the feasibility of the lyophilized powder dosage form of the compound formulation.

Example E0: Study on the druggability of the lyophilized dosage form:
To preliminarily study and explore the druggability of the lyophilized powder formulation, the factors that may affect the druggability such as excipients and pH were preliminarily screened.

Imiquimod was dissolved with hydrochloric acid or imiquimod hydrochloride was dissolved with pure water to obtain an aqueous solution of imiquimod hydrochloride, and then epirubicin hydrochloride was dissolved to obtain a solution containing effective components, wherein the mass ratio of the effective components epirubicin to imiquimod was 1:1 to 1:10; secondly, the pH value of the solution was attempted to be adjusted to observe whether the change in pH value affects the stability of the solution; subsequently, the lyoprotectants sucrose, lactose, mannitol or cyclodextrin (the concentration was between 10 mg/mL and 50 mg/mL) were added to observe the state of the samples, so as to determine if there was obvious instability, and the samples were lyophilized with lyophilization technology, wherein the mass fraction of the lyoprotectant was 65% to 96% after lyophilization, and the appearance of the lyophilized powder was observed to determine if there were shapeless, lumpy, caked or uneven samples that could be distinguished by naked eyes; finally, the lyophilized samples were reconstituted with a vehicle to observe if there was the phenomenon of insolubility and unevenness, and the pH values of the solutions were re-measured. When the concentration of epirubicin in the solutions was 1 mg/mL, the pH values were close to those before lyophilization, about 3.8 to 5.5.

In the preparation process of the formulation, the pH adjustment process and the addition of the lyoprotectant did not lead to obvious instability such as uneven solution or precipitation. After lyophilization, the samples all maintained good morphology, and were reconstituted successfully, and there was no phenomenon of insolubility or uneven dissolution. Therefore, the formula of the lyophilized powder formulation can be further studied on the stability to detect the change in impurity content.

### Example E1: Preliminary attempt of the effect of adding a lyoprotectant on the stability of the formulation

The liquid formulation was prepared according to the method of Example D1, wherein when the pH value was not adjusted, the initial pH value was about 3.0. In addition, sodium bicarbonate was used to adjust the pH value to 3.5, wherein the final concentrations of epirubicin and imiquimod were 1 mg/mL and 4 mg/mL, respectively. And different contents of lactose or mannitol were added before lyophilization (the concentrations at the time of preparation were 10 mg/mL, 25 mg/mL and 50 mg/mL, and the converted mass fractions were 67%, 83% and 91%). The impurity contents on day 0 after lyophilization were investigated. The results are shown in Table 5. The results have shown that the initial maximum unknown impurity contents in the compound formulation have exceeded the limits without adding any lyoprotectant, and the initial impurity content decreases after adding appropriate content of the lyoprotectant. The protective effect of lactose on the lyophilized formulation is better than that of mannitol. The increase of pH and the increase of lactose content are all favorable to the stability of the product.

**Table 5 The record table of the initial impurity contents of epirubicin (EPI) in the epirubicin/imiquimod lyophilized powder formulations after adding different contents of lactose or mannitol.**

| Sam ple | EPI-A (%) | EPI-C (%) | Maximu m unknown impurity (%) | Total impuri ty (%) | pH before lyophil ization | pH after lyophil ization | Content of lactose | Content of mannitol |
|---|---|---|---|---|---|---|---|---|
| 0-0 | 0.44 | 0.21 | 0.82 | 1.72 | 2.98 | 2.97 | 0 | 0 |
| 1-1 | 0.65 | 0.28 | 0.90 | 3.22 | 2.96 | 2.84 | 67% | |
| 2-1 | 0.28 | 0.26 | 0.34 | 1.13 | 3.5 | 3.70 | | |
| 3-1 | 0.32 | 0.26 | 0.41 | 1.54 | 2.96 | 3.01 | 83% | |
| 4-1 | 0.24 | 0.28 | 0.21 | 0.93 | 3.5 | 3.61 | | |
| 5-1 | 0.23 | 0.27 | 0.21 | 1.03 | 2.96 | 2.99 | 91% | |
| 6-1 | 0.19 | 0.28 | 0.16 | 0.73 | 3.5 | 3.55 | | |
| 7-1 | 0.98 | 0.26 | 1.06 | 2.88 | 2.96 | 3.53 | | 67% |
| 8-1 | 0.47 | 0.29 | 0.38 | 1.41 | 3.5 | 3.86 | | |
| 9-1 | 0.89 | 0.28 | 0.68 | 2.35 | 2.96 | 3.32 | | 83% |
| 10-1 | 0.77 | 0.28 | 0.65 | 1.93 | 3.5 | 3.74 | | |
| 11-1 | 0.70 | 0.29 | 0.50 | 1.90 | 2.96 | 3.31 | | 91% |
| 12-1 | 0.34 | 0.29 | 0.43 | 1.23 | 3.5 | 3.61 | | |
| **Limi t** | **1.0** | **1.0** | **0.5** | **2.5** | **NA** | | | |

Example E2: Stability of the lyophilized powder formulation standing at 25°C for 5 days with lactose as the lyoprotectant.

To further explore the effect of the content of the lyoprotectant and initial pH on the stability of the lyophilized powder, the samples No.4-1, No.5-1 and No.6-1 in Example E1 were placed at 25°C, and the pH values and impurity contents of the samples were detected after reconstitution on day 5. The data list was shown in Table 6 with that of day 0. From the change in impurity content, the change in the impurity content of the lyophilized powder formulation with initial pH value of 3.5 is better than that of lyophilized powder formulation with pH value of 3.0, and also, when lactose content is 91%, the increase of the impurity content is the least.

**Table 6 The record table of the initial impurity contents of epirubicin (EPI) in the compound lyophilized powder formulations of epirubicin/imiquimod added with lactose and the impurity contents of epirubicin (EPI) after being placed at 25°C for 5 days.**

| Na me | Initial pH | Time (days) | EPI-A (%) | EPI-C (%) | Maximu m unknow n impurity (%) | Total impurity (%) | pH value | Content of lactose |
|---|---|---|---|---|---|---|---|---|
| 4-1 | 3.5 | 0 | 0.24 | 0.28 | 0.21 | 0.93 | 3.61 | 83% |
| | | 5 | 0.52 | 0.29 | 0.19 | 1.55 | 3.56 | |
| 5-1 | 3.0 | 0 | 0.23 | 0.27 | 0.21 | 1.03 | 2.99 | 91% |
| | | 5 | 0.68 | 0.28 | 0.41 | 2.17 | 2.94 | |
| 6-1 | 3.5 | 0 | 0.19 | 0.28 | 0.16 | 0.73 | 3.55 | 91% |
| | | 5 | 0.35 | 0.28 | 0.15 | 1.00 | 3.48 | |
| **Limit** | **1.0** | **1.0** | **0.5** | **2.5** | **NA** | | | |

### Example E3: Screening of different lyoprotectants

Based on the preliminary exploration of the lyoprotectant effect and the attempt of the protectant content, the effect of different lyoprotectants on the stability of the lyophilized powder formulation under the same content was further explored. Based on the relationship between the change trend of pH value and the change trend of impurity, the stability of the lyophilized formulation at higher pH values was further attempted.

Lactose (disaccharide), mannitol (non-saccharide), cyclodextrin (cyclic oligosaccharide) and glucose (monosaccharide) were selected from the commonly used lyoprotectants for injections to verify their protective effects on lyophilized powder formulations at the lyoprotectant usage of 91 %.

First, the liquid formulation was prepared according to the method of Example D1, the pH of the solution was adjusted to 3.5 or 4.0, and different lyoprotectants were added respectively, wherein the final concentration of imiquimod was 4 mg/mL, the concentration of epirubicin was 1 mg/mL, and the final content of lyoprotectants after lyophilization was 91%. The change in the impurity content of epirubicin hydrochloride was detected after it was placed at 40°C for 5 days, and the results are shown in Table 5. In Table 7, the lyophilized powder formulation with initial pH of 4.0 has the best stability when the lyoprotectant is lactose, the impurity contents of which remain at a low level after being placed at 40°C for 5 days, with no significant increase in impurities, and the impurity growth is significant under the condition of pH=3.5; and for other lyoprotectants, the impurity growth is obviously at both pH 3.5 and pH 4.0. Therefore, lactose is preferred as the lyoprotectant.

**Table 7 Epirubicin/imiquimod compound lyophilized powder formulations with different initial pH values added with different lyoprotectants. The record table of the initial impurity contents of epirubicin and the impurity contents of epirubicin (EPI) after being placed at 40°C for 5 days.**

| Lyoprotectant and content thereof | pH | Time (days) | EPI-A (%) | EPI-C (%) | Unknown single impurity (%) | Total impurity (%) |
|---|---|---|---|---|---|---|
| Lactose 91% | 3.5 | 0 | 0.24 | 0.26 | 0.13 | 0.72 |
| | | 5 | 2.27 | 0.22 | 1.56 | 5.18 |
| | 4.0 | 0 | 0.21 | 0.31 | 0.16 | 0.84 |
| | | 5 | 0.35 | 0.24 | 0.13 | 0.90 |
| Mannitol 91% | 3.5 | 0 | 0.34 | 0.29 | 0.43 | 1.23 |
| | | 5 | 11.49 | 0.06 | 1.17 | 15.69 |
| | 4.0 | 0 | 0.32 | 0.31 | 0.23 | 1.01 |
| | | 5 | 7.02 | 0.22 | 0.86 | 9.63 |
| Cyclodextrin 91% | 3.5 | 0 | 0.51 | 0.30 | 0.14 | 1.22 |
| | | 5 | 5.20 | 0.26 | 2.46 | 9.72 |
| | 4.0 | 0 | 0.34 | 0.31 | 0.14 | 0.95 |
| | | 5 | 1.18 | 0.30 | 0.24 | 2.09 |
| Glucose 91% | 3.5 | 0 | 0.33 | 0.31 | 0.19 | 1.06 |
| | | 5 | 8.29 | 0.22 | 3.65 | 16.02 |
| | 4.0 | 0 | 0.31 | 0.31 | 0.17 | 1.02 |
| | | 5 | 1.20 | 0.29 | 0.75 | 3.19 |
| **Limit** | | | **1.0** | **1.0** | **0.5** | **2.5** |

### Example E4: Effect of the content of lactose on the stability of a lyophilized powder formulation

Based on the results of the previous examples, the effect of adding different contents of lactose on the impurity content of the lyophilized powder formulation was investigated when the pH value was 4.0.

According to the preparation method described in Example D1, the mixed injection of EPI and R837 was prepared. After adding different contents of the lyoprotectant lactose, the pH was adjusted to 4.0 with sodium bicarbonate, wherein the final concentration of imiquimod was 4 mg/mL, the concentration of epirubicin was 1 mg/mL, the concentrations of the lyoprotectants were 20 mg/mL, 30 mg/mL, 40 mg/mL and 50 mg/mL, respectively, and the corresponding lyoprotectant mass fractions after lyophilization were 80%, 86%, 89% and 91%, respectively. And the impurity contents were detected on the day after lyophilization, on day 5 and day 13 after it was placed at 40°C. The results are shown in Table 8. With the increase of the initial content of the lyoprotectant, the increase of the impurity contents of different compound formulations placed under the condition of the same pH and at the same temperature slows down, indicating that their chemical stability increases. When the content of the lyoprotectant is 80%, 86%, 89% and 91%, all of the impurity contents do not exceed the limit, and the chemical stability is the best when the initial content of the lyoprotectant is 91%.

**Table 8 The record table of the initial impurity contents of epirubicin (EPI) in the reconstituted solutions (pH 4.0) of the compound lyophilized powder formulations of epirubicin/imiquimod added with different contents of lactose and the impurity contents on day 5 and day 13 after being placed at 40°C.**

| Content of lyoprotectant lactose | Time (days) | EPI-A (%) | EPI-C (%) | Unknown single impurity (%) | Total impurity (%) |
|---|---|---|---|---|---|
| 80% | 0 | 0.26 | 0.29 | 0.14 | 0.97 |
| | 5 | 0.36 | 0.32 | 0.15 | 1.29 |
| | 13 | 0.50 | 0.29 | 0.13 | 1.53 |
| 86% | 0 | 0.29 | 0.30 | 0.14 | 0.99 |
| | 5 | 0.29 | 0.29 | 0.14 | 0.87 |
| | 13 | 0.41 | 0.29 | 0.13 | 1.34 |
| 89% | 0 | 0.28 | 0.31 | 0.15 | 0.91 |
| | 5 | 0.29 | 0.28 | 0.14 | 0.84 |
| | 13 | 0.35 | 0.29 | 0.13 | 1.17 |
| 91% | 0 | 0.21 | 0.31 | 0.16 | 0.84 |
| | 5 | 0.35 | 0.24 | 0.13 | 0.90 |
| | 13 | 0.32 | 0.26 | 0.12 | 1.09 |
| **Limit** | | **1.0** | **1.0** | **0.5** | **2.5** |

Example E5: Preparing formulation samples under different pH conditions and investigating the stabilities of lyophilized formulations.

Liquid formulations with pH values of 3.5, 4.0, 4.5 and 5.0 respectively were prepared by the method of Example D1, wherein the final concentration of imiquimod was 4 mg/mL, and the concentration of epirubicin was 1 mg/mL. After pre-freezing, the liquid formulations were lyophilized, left at different temperatures for a certain period of time, and then the lyophilized powder was reconstituted, and the impurity content was detected. The experimental conditions and results are shown in Table 9. Different from the change trend of the total impurity of epirubicin hydrochloride in injection forms, when the initial pH value increases, the increase of total impurity slows down. When the lyoprotectant is lactose and the content is 91%, the changes in the unknown single impurity and the total impurity are all lower than that of injection forms, that is, under the conditions of the same pH (pH=5.0) and temperature (25°C), the impurity contents of lyophilized powder formulations stored for a longer time (30 days) are much lower than that of injection dosage forms stored for a short time (5 days), which meets the current national Pharmacopoeia standards. Wherein, when the pH before lyophilization is 3.5, the impurities in the sample on day 5 after being at 40°C have exceeded the limits specified in the Pharmacopoeia, so the sample under this condition was not analyzed for 30-day impurities.

**Table 9 The record table of the impurity contents of epirubicin (EPI) in the epirubicin/imiquimod lyophilized powder formulations at different initial pH values after being placed in different temperature environments for different time.**

| Initial pH | Temperature (°C) | Time (days) | EPI-A (%) | EPI-C (%) | Unknown single impurity (%) | Total impurity (%) |
|---|---|---|---|---|---|---|
| 3.5 | - | 0 | 0.24 | 0.26 | 0.13 | 0.72 |
| | 25 | 30 | 0.85 | 0.25 | 0.28 | 1.77 |
| | 40 | 4 | 1.34 | 0.28 | 0.61 | 2.92 |
| | | 30 | - | - | - | - |
| 4.0 | - | 0 | 0.21 | 0.31 | 0.16 | 0.84 |
| | 25 | 31 | 0.29 | 0.27 | 0.16 | 0.87 |
| | 40 | 4 | 0.32 | 0.31 | 0.13 | 1.05 |
| | | 28 | 0.39 | 0.28 | 0.19 | 1.28 |
| 4.5 | - | 0 | 0.22 | 0.29 | 0.18 | 0.93 |
| | 25 | 31 | 0.27 | 0.26 | 0.19 | 0.93 |
| | 40 | 4 | 0.26 | 0.28 | 0.19 | 1.05 |
| | | 28 | 0.22 | 0.29 | 0.27 | 1.26 |
| 5.0 | - | 0 | 0.20 | 0.32 | 0.18 | 0.88 |
| | 25 | 30 | 0.27 | 0.25 | 0.22 | 0.97 |
| | 40 | 4 | 0.28 | 0.26 | 0.25 | 1.06 |
| | | 30 | 0.23 | 0.31 | 0.30 | 1.30 |
| **Limit** | | | **1.0** | **1.0** | **0.5** | **2.5** |

Example E6: Varying the ratio of the two components, and studying the change in the impurity contents in lyophilized formulations.

Liquid formulations with pH values of 3.5, 4.0 and 4.5 respectively were prepared by the method of Example D1, and lactose was added, wherein the final concentration of imiquimod was 2 mg/mL and the concentration of epirubicin was 1 mg/mL. The contents of lactose are shown in Table 10. The impurity contents of epirubicin on the day after lyophilization and 27 days after storing at 40°C were recorded. The record was as shown in Table 10. The results have shown that with the increase of the initial pH and the increase of the lactose content, the impurity content of the sample decreases when it is placed at 40°C for 27 days. When the initial pH is 4.0 and the lactose content in the sample is 67%, the total impurity contents exceed the limits on day 27, and the impurity contents are all below the limits under other conditions. When pH=4.5, several lactose contents can reduce the increase of the impurity content in the sample after lyophilization.

**Table 10 The record table of the impurity contents of epirubicin (EPI) in the epirubicin/imiquimod complex compound lyophilized powder formulations at different initial pH values when they were placed in an environment of 40°C for 27 days.**

| Content of lactose | Initial pH value | Time of placement (days) | EPI-A (%) | EPI-C (%) | Maximum unknown impurity (%) | Total impurity (%) |
|---|---|---|---|---|---|---|
| 67% | 3.5 | 0 | 0.62 | 0.32 | 0.20 | 1.51 |
| | | 27 | 4.89 | 0.22 | 7.20 | 16.99 |
| | 4.0 | 0 | 0.50 | 0.27 | 0.15 | 1.08 |
| | | 27 | 0.87 | 0.27 | 0.62 | 2.51 |
| | 4.5 | 0 | 0.42 | 0.31 | 0.23 | 1.21 |
| | | 27 | 0.38 | 0.28 | 0.21 | 1.18 |
| 83% | 3.5 | 0 | 0.41 | 0.30 | 0.15 | 1.09 |
| | | 27 | 2.67 | 0.24 | 4.24 | 9.76 |
| | 4.0 | 0 | 0.44 | 0.33 | 0.16 | 1.18 |
| | | 27 | 0.73 | 0.29 | 0.33 | 1.90 |
| | 4.5 | 0 | 0.47 | 0.32 | 0.20 | 1.18 |
| | | 27 | 0.41 | 0.28 | 0.18 | 1.15 |
| 91% | 3.5 | 0 | 0.45 | 0.31 | 0.14 | 1.08 |
| | | 27 | 1.71 | 0.28 | 2.40 | 6.02 |
| | 4.0 | 0 | 0.37 | 0.30 | 0.15 | 1.00 |
| | | 27 | 0.52 | 0.29 | 0.14 | 1.33 |
| | 4.5 | 0 | 0.40 | 0.33 | 0.19 | 1.08 |
| | | 27 | 0.37 | 0.29 | 0.18 | 1.07 |
| **Limit** | | | **1.0** | **1.0** | **0.5** | **2.5** |

### Example E7: Further screening for the optimal pH

The mixed solution of epirubicin and imiquimod was prepared according to the method of Example D1, the pH of which was adjusted to 3.8, 4.0, 4.2 and 4.4, and lactose was added, wherein the ratio of epirubicin to imiquimod in the samples of two specifications was 1: 2 or 1:4, the final concentration of the lyoprotectant was 91%, and the corresponding mass fractions in the two specifications were 94% and 91% respectively. After lyophilization, the samples were placed under the condition of 40°C, and the impurity contents in the sample were detected at different time points. The results are shown in Table 11.

The results have shown that no matter what concentration ratio is, the growth trend of impurities in epirubicin is related to the initial pH value. The higher the pH value is, the more stable the formulation is. When the pH value is 3.8, the content of the maximum unknown impurity exceeds the limit, and when the pH value is above 3.8, the requirements of impurity content stability are all met.

**Table 11 The record table of the initial impurity contents of epirubicin in the epirubicin/imiquimod complex compound lyophilized powder formulations at different initial pH values and impurity contents of epirubicin (EPI) when they were placed in an environment of 40°C for 19 days and 30 days.**

| EPI: R837 | Cont ent of lacto se | Initi al pH | Time of place ment (days) | pH after reconst itution | EPI-A (%) | EPI-C (%) | Maxim um unkno wn impurit y (%) | Total impurit y (%) |
|---|---|---|---|---|---|---|---|---|
| 1:2 | 94% | 3.8 | 0 | 3.87 | 0.21 | 0.26 | 0.09 | 0.61 |
| | | | 19 | 3.88 | 0.55 | 0.28 | 0.33 | 1.54 |
| | | | 30 | 3.86 | 0.67 | 0.28 | 0.52 | 2.17 |
| | | 4.0 | 0 | 4.04 | 0.20 | 0.26 | 0.09 | 0.60 |
| | | | 19 | 4.12 | 0.33 | 0.28 | 0.10 | 1.01 |
| | | | 30 | 4.07 | 0.41 | 0.26 | 0.14 | 1.10 |
| | | 4.2 | 0 | 4.23 | 0.20 | 0.26 | 0.09 | 0.61 |
| | | | 19 | 4.26 | 0.30 | 0.30 | 0.11 | 0.80 |
| | | | 30 | 4.26 | 0.24 | 0.28 | 0.10 | 0.87 |
| | | 4.4 | 0 | 4.38 | 0.21 | 0.26 | 0.10 | 0.57 |
| | | | 19 | 4.47 | 0.22 | 0.28 | 0.12 | 0.72 |
| | | | 30 | 4.45 | 0.24 | 0.28 | 0.12 | 0.76 |
| 1:4 | 91% | 3.8 | 0 | 3.85 | 0.25 | 0.27 | 0.11 | 0.84 |
| | | | 19 | 3.89 | 0.42 | 0.26 | 0.17 | 1.33 |
| | | | 30 | 3.91 | 0.47 | 0.26 | 0.28 | 1.64 |
| | | 4.0 | 0 | 4.05 | 0.19 | 0.27 | 0.12 | 0.94 |
| | | | 19 | 4.08 | 0.26 | 0.28 | 0.12 | 0.96 |
| | | | 30 | 4.07 | 0.30 | 0.25 | 0.11 | 1.01 |
| | | 4.2 | 0 | 4.26 | 0.15 | 0.27 | 0.14 | 0.91 |
| | | | 19 | 4.25 | 0.23 | 0.28 | 0.13 | 0.90 |
| | | | 30 | 4.28 | 0.21 | 0.29 | 0.13 | 0.86 |
| | | 4.4 | 0 | 4.45 | 0.22 | 0.30 | 0.15 | 1.15 |
| | | | 19 | 4.43 | 0.22 | 0.30 | 0.15 | 1.03 |
| | | | 30 | 4.44 | 0.20 | 0.26 | 0.15 | 0.88 |
| **Limit** | | | | | **1.0** | **1.0** | **0.5** | **2.5** |

### Example F Investigation of the stabilities of mixed lyophilized powder formulations of different anthracycline-based drugs and imidazoquinoline-based immunomodulators at pH=4.0

### Example F1: Stabilities of mixed lyophilized powder formulations of epirubicin hydrochloride and resiquimod (R848)

Firstly, a mixed formulation was prepared. Specifically, R848 was dissolved with hydrochloric acid to obtain a hydrochloride solution of R848, and epirubicin hydrochloride was further added, with the mass ratio of R848 to epirubicin was 4:1. The lyoprotectant lactose was added, with the final content of lactose was 91%. Lyophilization was performed, and the lyophilized powder was placed at different ambient temperatures to investigate the stability. On the day 5 and day 10, samples were taken to detect the change in the impurity contents of the samples.

Different from R837 mixed with epirubicin, after R848 was dissolved with hydrochloric acid and epirubicin was added, the initial pH of the solution system was about 5.5, which was higher than that of imiquimod hydrochloride. Therefore, the pH of the system was adjusted to about 4.0 by using an acidic solution. The changes in impurity under the conditions of the original pH (pH=5.5) and pH=4.0 were detected. The impurity detection results of R848 are shown in Table 12. As there is no R848 drug on the market at present, it is impossible to determine the stability of R848 from the limit of the impurity content. However, it can be seen from the data in Table 12 that the impurity content of R848 has hardly changed with time, whether under the condition of 25°C or under the condition of 40°C, thus it can be determined that R848 has a good stability in the mixed lyophilized powder formulation.

**Table 12 The record table of the initial impurity contents of R848 in the mixed lyophilized powder formulations of epirubicin and resiquimod (R848) under different pH conditions and impurity contents of R848 in the samples when they stood under temperature conditions for 5 days and 10 days.**

| Lyoprotectant and content thereof | pH | Temperature (°C) | Time (days) | Maximum unknown impurity (%) | Total impurity (%) |
|---|---|---|---|---|---|
| Lactose 91% | 5.5 | \ | 0 | 0.27 | 0.58 |
| | 4.0 | | | 0.27 | 0.59 |
| | 5.5 | 25 | 5 | 0.26 | 0.58 |
| | 4.0 | | | 0.27 | 0.60 |
| | 5.5 | | 10 | 0.27 | 0.58 |
| | 4.0 | | | 0.27 | 0.59 |
| | 5.5 | 40 | 5 | 0.26 | 0.58 |
| | 4.0 | | | 0.26 | 0.58 |
| | 5.5 | | 10 | 0.27 | 0.58 |
| | 4.0 | | | 0.26 | 0.57 |

The impurity detection results of epirubicin are shown in Table 13. When the pH of the system before lyophilization is not adjusted, i.e., the initial pH is 5.5, the maximum unknown impurity content of epirubicin increases after mixing, which exceeds the limits specified by the standard. In the subsequent stability experiments, the maximum unknown impurity content is still increasing slowly, which does not meet the quality requirements of the formulation. When the initial pH value is adjusted to 4.0, the initial content of the maximum unknown impurity is greatly reduced. As time goes on, the impurity content does not increase greatly under different temperature conditions, and it has been always within the limit, which shows that the mixed lyophilized powder formulation has a good stability under the condition of pH=4.0.

**Table 13 The record table of the initial impurity contents of epirubicin in the mixed lyophilized powder formulations of epirubicin and R848 under different pH conditions and impurity contents of epirubicin when they stood under different temperature conditions for 5 days and 10 days.**

| Lyoprotectant and content thereof | pH | Temperature (°C) | Time (days) | EPI-A (%) | EPI-C (%) | Maximum unknown impurity (%) | Total impurity (%) |
|---|---|---|---|---|---|---|---|
| Lactose 91% | 5.5 | \ | 0 | 0.29 | 0.26 | 0.95 | 2.30 |
| | 4.0 | | | 0.30 | 0.26 | 0.26 | 1.23 |
| | 5.5 | 25 | 5 | 0.28 | 0.26 | 1.07 | 2.42 |
| | 4.0 | | | 0.30 | 0.26 | 0.26 | 1.24 |
| | 5.5 | | 10 | 0.28 | 0.23 | 1.05 | 2.43 |
| | 4.0 | | | 0.29 | 0.23 | 0.25 | 1.22 |
| | 5.5 | 40 | 5 | 0.29 | 0.26 | 0.99 | 2.34 |
| | 4.0 | | | 0.33 | 0.26 | 0.26 | 1.30 |
| | 5.5 | | 10 | 0.28 | 0.23 | 1.11 | 2.48 |
| | 4.0 | | | 0.31 | 0.24 | 0.25 | 1.26 |
| **Limit** | | | | **1.0** | **1.0** | **0.5** | **2.5** |

Example F2: Investigation of the stabilities of mixed lyophilized powder formulations of doxorubicin (ADM) and imiquimod.

The mixed liquid formulation of doxorubicin and imiquimod was prepared by the method of Example D1. The initial pH of doxorubicin hydrochloride mixed with imiquimod was 3.2, and the adjusted pH was 4.0. The difference of the chemical stabilities of lyophilized powder formulations obtained after lyophilization at two pH values was explored. The lyoprotectant was lactose, and the content was 91%. It has been found in the test that imiquimod is very stable in the mixed formulation, the impurity content of which is substantially unchanged. Impurity B 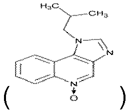 is kept at about 0.05%, Impurity A 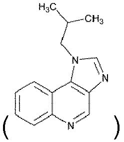 is kept at about 0.05%, and Impurity C 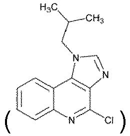 is not detected. The maximum unknown single impurity is kept at about 0.18%, and the total impurity is substantially within 0.39%. Imiquimod impurities only fluctuate slightly when the formulation is placed at different temperatures for different time, none of which exceeds the limits specified in the R837 formulation standard: Impurity A 0.15%, Impurity B 0.20%, Impurity C 0.15%, maximum unknown single impurity 0.20% and total impurity 1.0%.

The impurity contents of doxorubicin in the stability investigation are recorded in Table 14. According to the guidance of the United States Pharmacopoeia, Impurity A of doxorubicin is 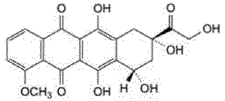 and Impurity C is 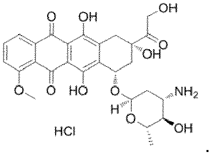 The impurities of doxorubicin also increase rapidly at low pH. When the pH is adjusted to 4.0, the impurities can be kept at safe levels and the stability is better.

**Table 14 The record table of the initial impurity contents in the mixed lyophilized powder formulations of doxorubicin hydrochloride (ADM) and imiquimod (R837) under different pH conditions and the impurity contents of doxorubicin when they stood under different temperature conditions for 5 days and 10 days**

| Lyoprotectant and content thereof | pH | Temperature (°C) | Time (days) | Impurity A (%) | Maximum unknown impurity (%) | Total impurity (%) |
|---|---|---|---|---|---|---|
| Lactose, 91% | 3.2 | - | 0 | 0.37 | 0.07 | 0.58 |
| | 4.0 | | | 0.22 | 0.07 | 0.33 |
| | 3.2 | 25 | 5 | 0.84 | 0.27 | 1.35 |
| | 4.0 | | | 0.25 | 0.07 | 0.36 |
| | 3.2 | | 10 | 0.80 | 0.36 | 1.54 |
| | 4.0 | | | 0.32 | 0.07 | 0.46 |
| | 3.2 | 40 | 5 | 2.13 | 1.33 | 4.41 |
| | 4.0 | | | 0.33 | 0.07 | 0.48 |
| | 3.2 | | 10 | 2.69 | 1.67 | 5.61 |
| | 4.0 | | | 0.26 | 0.07 | 0.45 |
| **Limit** | | | | **1.0** | **0.5** | **3.0** |

### Example F3: Investigation of the stabilities of compound lyophilized powder formulations of mitoxantrone hydrochloride and imiquimod

The mixed liquid formulation of mitoxantrone hydrochloride and imiquimod was prepared by the method of Example D1. After adding lyoprotectant, lyophilization was performed to prepare a lyophilized powder formulation, in which the content of lactose was 91%. The chemical stability of the lyophilized powder was tested when pH=3.2 before lyophilization (without pH adjustment) and pH=4.0 (after adjustment). Wherein, the stability of imiquimod was consistent with the phenomenon of the above mixed formulation, and the impurities related to imiquimod had no obvious change and were all within the limits. Impurity A of mitoxantrone hydrochloride is 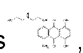 and Impurity D is 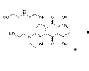 . The impurity contents of mitoxantrone hydrochloride are shown in Table 11. According to the guidance of the United States Pharmacopoeia, Impurity B and Impurity C are not detected. The maximum unknown single impurity content of mitoxantrone hydrochloride should not exceed 1.0%, and the total impurity content should not exceed 3.0%. In the impurity content record Table 15, no matter at low pH or high pH, the impurity limits are not exceeded. However, compared with the original pH after dissolution, the increase in impurity is slower after adjusting the pH value to 4.0, which substantially fluctuates around the initial impurity content, indicating that the lyophilized powder formulation is more stable when pH=4.0.

**Table 15 The record table of the initial impurity contents of mitoxantrone hydrochloride in the mixed lyophilized powder formulations of mitoxantrone hydrochloride and R837 under different pH conditions and impurity contents of mitoxantrone hydrochloride under the conditions of different temperatures**

| Lyoprotectan t and content thereof | pH | Temperatur e (°C) | Time (days ) | Impurit y A (%) | Impurit y D (%) | Maximu m unknown impurity (%) | Total impurit y (%) |
|---|---|---|---|---|---|---|---|
| Lactose, 91% | 3. 2 | - | 0 | 0.14 | 0.06 | 0.13 | 0.56 |
| | 4. 0 | | | 0.14 | 0.06 | 0.13 | 0.57 |
| | 3. 2 | 25 | 5 | 0.14 | 0.06 | 0.13 | 0.61 |
| | 4. 0 | | | 0.14 | 0.07 | 0.13 | 0.56 |
| | 3. 2 | | 10 | 0.14 | 0.06 | 0.15 | 0.61 |
| | 4. 0 | | | 0.13 | 0.06 | 0.15 | 0.60 |
| | 3. 2 | 40 | 5 | 0.15 | 0.07 | 0.13 | 0.66 |
| | 4. 0 | | | 0.14 | 0.06 | 0.14 | 0.57 |
| | 3. 2 | | 10 | 0.15 | 0.07 | 0.15 | 0.71 |
| | 4. 0 | | | 0.14 | 0.07 | 0.15 | 0.55 |
| **Limit** | | | | **1.0** | **1.0** | **1.0** | **3.0** |

### Example F4: Investigation of the stabilities of mixed lyophilized powder formulations of pirarubicin and imiquimod

The mixed lyophilized powder formulation of pirarubicin and imiquimod was prepared. The pH of the formulation before lyophilization was 4.0 or 5.0, and the lyoprotectant was lactose, with the content of 91%. The impurity contents of imiquimod and pirarubicin after lyophilization were detected, and the results are shown in Table 16 and Table 17. The results have shown that the impurity content of imiquimod does not change greatly under different pH conditions, indicating that imiquimod is more stable in the system. Under the condition of pH=4.0, the impurity growth of pirarubicin is greater than that under the condition of pH=5.0 (measured pH=4.99). The detection limit of drug impurities of pirarubicin is not specified in the Pharmacopoeia, thus it can only be determined by the impurity growth trend, and the condition of pH=5.0 is more favorable to the stability of the formulation.

**Table 16 The record table of the initial impurity contents of imiquimod in the mixed lyophilized powder formulations of pirarubicin hydrochloride and imiquimod under the different pH conditions and the impurity contents of imiquimod when they were placed at 40°C for 5 days and 15 days.**

| Lyoprotectant and content thereof | pH | Temperature (°C) | Time (days) | R837-A (%) | R837-B (%) | Maximum unknown impurity (%) | Total impurity (%) |
|---|---|---|---|---|---|---|---|
| Lactose, | 4.99 | 40 | 0 | 0.05 | 0.04 | 0.13 | 0.29 |
| 91% | 4.0 | | | 0.05 | 0.04 | 0.13 | 0.29 |
| | 4.99 | | 5 | 0.05 | 0.04 | 0.13 | 0.29 |
| | 4.0 | | | 0.05 | 0.04 | 0.14 | 0.30 |
| | 4.99 | | 15 | 0.05 | 0.04 | 0.13 | 0.29 |
| | 4.0 | | | 0.05 | 0.04 | 0.13 | 0.28 |
| **Limit** | | | | **0.15** | **0.20** | **0.20** | **1.0** |

**Table 17 The record table of the initial impurity contents of pirarubicin hydrochloride in the mixed lyophilized powder formulations of pirarubicin hydrochloride and imiquimod under the different pH conditions and the impurity contents of pirarubicin hydrochloride when they were placed at 40°C for 5 days and 15 days.**

| Lyoprotectant and content thereof | Temperature (°C) | Time (days) | pH | Maximum unknown impurity (%) | Total impurity (%) |
|---|---|---|---|---|---|
| Lactose, 91% | 40 | 0 | 4.99 | 0.28 | 1.28 |
| | | | 4.0 | 0.29 | 1.04 |
| | | 5 | 4.99 | 0.28 | 1.28 |
| | | | 4.0 | 1.51 | 2.86 |
| | | 15 | 4.99 | 0.54 | 1.93 |
| | | | 4.0 | 1.50 | 3.27 |

### Example G Animal experiments

Example G1: Application of epirubicin used in combination with R837 in the treatment of bladder cancer.

The mixed lyophilized powder formulation of epirubicin and R837 was prepared for later use.

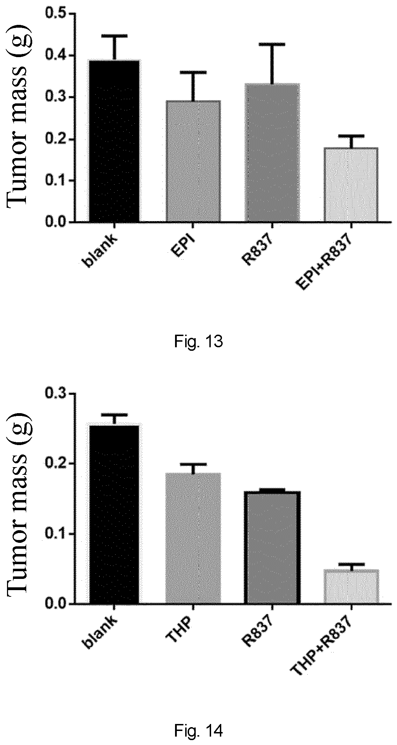

Tumor model of MB49 bladder cancer in situ in mice were established and randomly divided into the following 4 groups:
Blank: perfusion of 5% glucose solution;
EPI: perfusion of the epirubicin solution dissolved in 5% glucose solution;
R837: perfusion of the imiquimod suspension dispersed in 5% glucose solution;
EPI+R837: perfusion of the mixed lyophilized powder formulation of epirubicin and imiquimod dissolved in 5% glucose solution;
the first bladder perfusion administration was recorded as day 0, the second administration was carried out on day 7, and the mice were sacrificed on day 12. Bladder tumors were taken, the mass was recorded, and the average of tumor mass in different groups was summarized. The results are shown in Fig. 13, wherein the tumor mass of mice treated with compound formulations was significantly lower than that of other groups, indicating that the mixed lyophilized powder formulation of epirubicin and imiquimod has the effect of inhibiting the growth of bladder cancer.

### Example G2: Application of pirarubicin (THP) used in combination with R837 in the treatment of bladder cancer.

The in-situ models of bladder cancer in mice were established by the same method as in Example G1, and the mixed lyophilized powder formulation of pirarubicin and imiquimod was prepared for later use. The mice were randomly divided into the following groups:
Blank: perfusion of 5% glucose solution;
THP: perfusion of the pirarubicin solution dissolved in 5% glucose solution;
R837: perfusion of the imiquimod suspension dispersed in 5% glucose solution;
THP+R837: perfusion of the mixed lyophilized powder formulation of pirarubicin and imiquimod dissolved in 5% glucose solution,
bladder perfusion treatment was performed, the treatment frequency and termination time of which were the same as Example D1. After two times of bladder perfusion treatment, the bladder cancer in the mice was obtained by dissection, and the tumor mass was recorded. The statistical chart is shown in Fig. 14. The compound formulation obtained by reconstituting with 5% glucose solution has an obvious anti-tumor effect. When pirarubicin or R837 is used alone, the tumor inhibition rates are 28.23% and 38.33%, respectively, while the mice treated with the compound formulation has a tumor inhibition rate as high as 81.6%. The Guinness formula q=E(A+B)/(EA+EB-EA*EB) is used to calculate the drug synergy, wherein E(A+B) is the tumor inhibition rate in the compound formulation treatment group, and EA and EB are the tumor inhibition rates when the two components are used respectively. When q≥1, indicating that the two ingredients have synergistic effects. q > 1 is obtained by formula calculation, indicating that the form of compound formulation can achieve the synergistic effect of anthracycline-based chemotherapy drugs and immunological adjuvants.

### Example G3: Application of doxorubicin (ADM) used in combination with resiquimod (R848) in the treatment of bladder tumor.

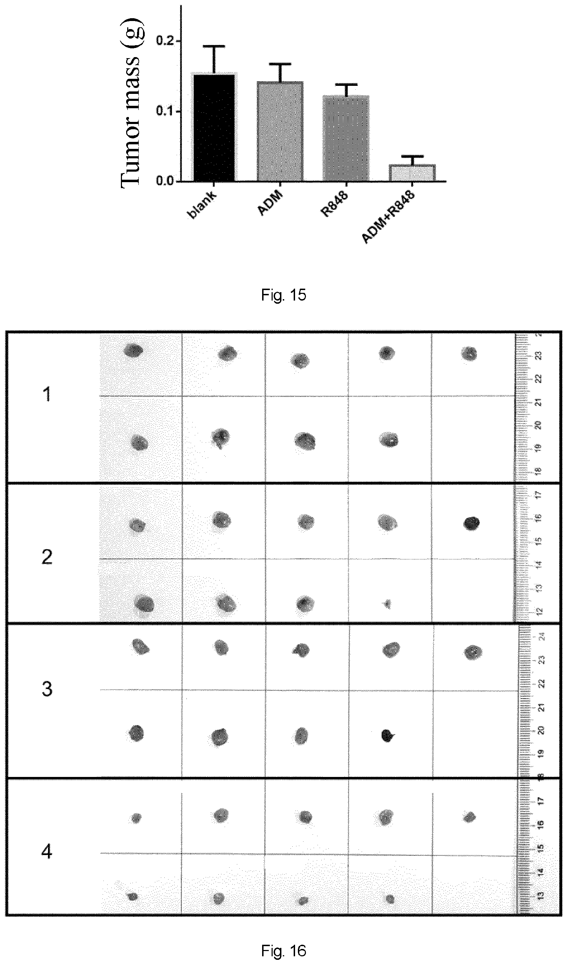

The in-situ models of bladder cancer in mice were established by the same method as in Example G1, the mixed lyophilized powder formulation of doxorubicin and imiquimod was prepared for later use, and the mice were randomly divided into groups (Blank: perfusion of 5% glucose solution; ADM: perfusion of the pirarubicin solution dissolved in normal saline; R848: perfusion of the resiquimod suspension dispersed in 5% glucose solution; ADM+R848: perfusion of the mixed lyophilized powder formulation of pirarubicin and resiquimod dissolved in 5% glucose solution), wherein hydroxypropyl methylcellulose was added as a penetration enhancer component before the compound formulation was used for bladder perfusion treatment. After two times of the bladder perfusion in each group, the bladder cancer in the mice was obtained by dissection, and the tumor mass was recorded. The statistical chart is shown in Fig. 15. Compared with the effects of Examples G1 and G2, the compound formulation obtained by reconstitution in normal saline has more obvious anti-tumor effect after being used in combination with a mucosal penetration enhancer. Wherein, in the group treated with the compound formulation, the tumor inhibition rate of mice is 85.1%, while in the group treated with R848 or doxorubicin alone, the tumor inhibition rates of mice are 21.8% and 8.6%, respectively, and the therapeutic effect of compound formulation is significantly enhanced.

Combining several sets of data in Example G, the compound lyophilized powder formulation of anthracycline-based chemotherapy drugs and imidazoquinoline-based immunological adjuvants can treat bladder tumor by bladder perfusion.

### Example H Tumor treatment experiments

### Example H1: Treatment of bladder cancer in situ

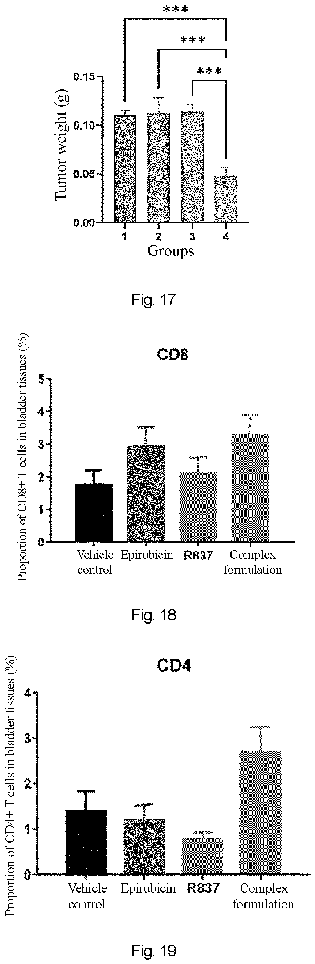

The in situ tumor models of bladder cancer in C57BL/6 mice were established. Specifically, on day 0, female C57BL/6 mice were inoculated with 2*10⁵ Luc-MB49 cells. On day 4 after inoculation, the tumor sizes were determined by in vivo fluorescence imaging, and mice with uniform tumor growth were selected and grouped. Nine mice in each group, a total of four groups, the grouping was as follows:
1: 100 µL of 5% glucose solution was perfused through bladders;
2: 100 µL of the epirubicin solution dissolved in 5% glucose solution was perfused through bladders;
3: 100 µL of the imiquimod hydrochlorid lyophilized powder dissolved in 5% glucose solution was perfused through bladders;
4: 100 µL of the lyophilized powder formulation described in the invention dissolved in 5% glucose solution was perfused through bladders;
wherein, the dosage of epirubicin was 60 µg/animal, and the dosage of R837 was 240 µg/animal. The bladder perfusion administration was performed on day 4 and day 11 after tumor inoculation. On day 3 after the second treatment, the mice were sacrificed, and the tumors in their bladders were taken and photographed. The photos of bladder tumors in different groups of mice are shown in Fig. 16. It can be seen by naked eyes that the bladder diameters of the mice in epirubicin+R837 group are the smallest, and the tumor sizes of other groups are uneven, and most tumors are larger than that of the mice in this group. The tumors were weighed, and the bladder tumor mass of the mice in different groups was summarized. The results are as shown in Fig. 17, in which the tumor mass of the mice in Group 4 is the smaller that is, the lyophilized powder formulation has the best therapeutic effect, which is consistent with the naked eye observation in Fig. 16.

### Example H2: Flow analysis of immune cells in lymph node and bladder.

The proportion and activity of immune cells in different samples in Example H1 were detected and analyzed by a flow cytometer.

The bladder and lymph node samples were homogenized respectively, centrifuged at 1800 rpm for 5 min. The supernatant and precipitate were separated, and digestive enzymes were added to the precipitate to prepare a tissue single cell suspension. Different immune cells were labeled by flow antibodies, and the contents of different immune cells were analyzed by a flow cytometer. The results are shown in Fig. 18 to Fig. 20. Fig. 18 shows the invasion of CD3⁺ and CD8⁺T cells in bladders. CD8⁺T cells are killer T cells, which have a direct killing effect on tumors. The existence of these cells shows that immunotherapy plays a role in tumor treatment, and the more CD8⁺T cells invade, the stronger the immunotherapy effect is. From the statistical chart, it can be seen that epirubicin leads to immune response in tumor sites through immunogenic death, the immune killing effect of which is further enhanced after being combined with R837, indicating that the bladder perfusion formulation of the invention has better immunotherapy potential.

Fig. 19 shows the invasion of CD3⁺ and CD4⁺T cells in tumors. On the one hand, CD4⁺T cells can stimulate the activation of CD8+T cells and maintain and strengthen the anti-tumor immune response. On the other hand, they can exert anti-tumor effect through IFN-γ mechanism. Therefore, the increase of CD4⁺T cell content in the initial stage of treatment is helpful to the anti-tumor immune response. As can be seen from the data in Fig. 19, the contents of CD4⁺T cells in the tumor sites in the treatment group of bladder perfusion with the formulation of the invention have increased by more than 2 times, indicating that the compound formulation of epirubicin of the invention can cause a stronger anti-tumor immune response.

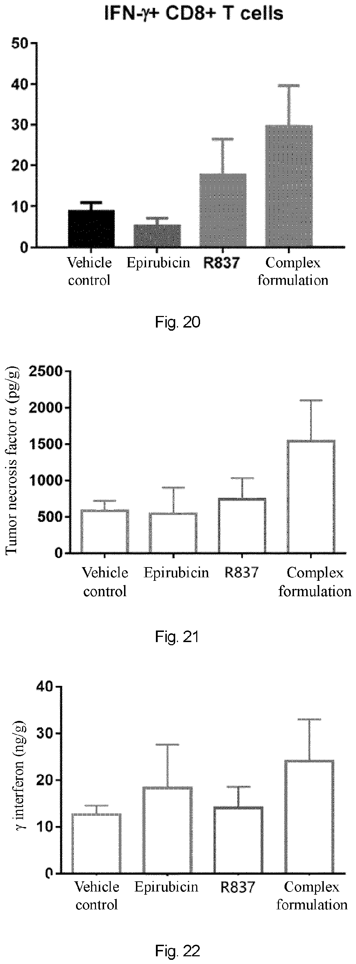

Fig. 20 shows the proportion of functional CD8⁺T cells after the CD8⁺T cells in the tumor sites being stimulated by a cell stimulating mixture. Cells with tumor killing potential in CD8⁺T cells will secrete IFN-γ intracellularly after being stimulated by the cell stimulating mixture. The ratio of cells with double positive signals to CD8 single positive cells was detected by labeling CD8 and intracellular IFN-γ by flow antibody staining. The results have shown that about 30% of CD8⁺T cells invaded in tumor sites of the mice treated with the lyophilized powder formulation described in the invention can accumulate IFN-γ within the cells after being stimulated, which is 1.5 to 6 times the proportions of corresponding cells in the single-component treatment group, indicating that the proportion of functional CD8⁺T cells in tumor sites is higher, and there is a greater immunotherapy potential, that is, the lyophilized powder formulation described in the invention has a stronger immunotherapy effect on bladder cancer.

Combining the flow analyses of several immune-related cells, it has been shown that bladder perfusion using a lyophilized powder formulation has a good effect of activating the anti-tumor immune response.

Example H3: Cytokine analysis of the supernatant obtained after centrifugation in Example C2. The contents of tumor necrosis factor (TNF-α) and interferon-γ (IFN-γ) in tumor tissues of different groups of mice were detected by using a cytokine detection kit, and the detection results are shown in Fig. 21 and Fig. 22. The higher the contents of TNF-α and IFN-γ are, the stronger the anti-tumor immune response in the tumor sites is. In contrast, the contents of two cytokines in the compound formulation group are relatively higher, especially the content of TNF-α. As shown in Fig. 21, the content of TNF-α in the compound formulation group is 2 to 3 times that in other groups, which proves that the mixed formulation has the effect of significantly enhancing the anti-tumor immune response. In Fig. 22, the IFN-γ content of the compound formulation group is higher than any other group, which is about 1.5 to 2 times that of other groups.

### Example I Verification of the effect of a mucosal permeability enhancer on drug retention in bladders

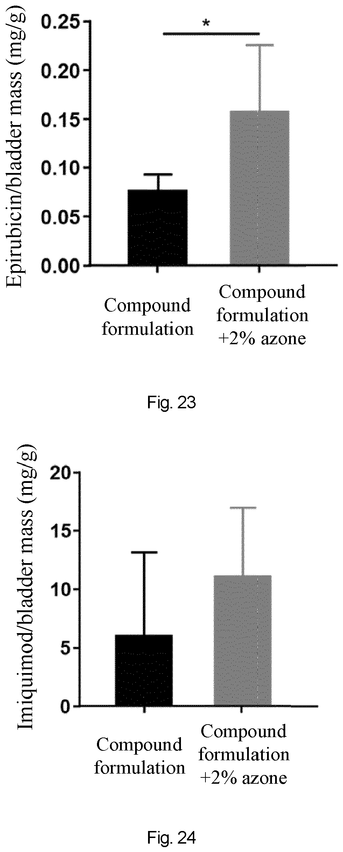

Complex formulation: 100 µL of the compound formulation of epirubicin and imiquimod was perfused into bladders;
Azone + complex formulation: A compound lyophilized powder formulation containing 2% (w/w) azone was prepared, and an amount of 100 µL was perfused after reconstitution into bladders;
the mixed liquid above was directly used for bladder perfusion in mice, and the perfusion process was carried out in mice under anesthesia. The perfusate was kept in the bladder for 1 hour, then the liquid was discharged. The mice were allowed to be out of anesthesia, helped to drink 500 µL of water by gavage to promote defecation. The bladder samples of the mice were obtained by dissection 6 hours after the drug was discharged, and each sample was weighed and recorded. The samples were grounded in the same volume of a buffer solution, and the effective components were extracted with methanol and an extraction solution. The supernatant was obtained by centrifugation. Then, the contents of the extracted effective components in different samples were detected by high performance liquid chromatography, and the relative contents per gram of the tissues were calculated. The results are shown in Fig. 23 and Fig. 24. Fig. 23 is a statistical graph of the effect of the mucosal penetration enhancer on the retention of epirubicin in bladders, and the tissue relative content of epirubicin can be seen; Fig. 24 is a statistical graph of the effect of the mucosal penetration enhancer on the retention of imiquimod in bladders, and the relative tissue content of imiquimod can be seen. Wherein, the compound formulation added with the azone component can significantly enhance the retention of drugs in bladder tissues, the retention amount of which is more than 2 times, indicating that the addition of penetration enhancers can help the retention of effective components, and further has the effect of increasing therapeutic effects.

The above description of the disclosed examples enables those skilled in the art to achieve or use the invention. Many modifications to these examples will be obvious to those skilled in the art. The invention is not limited to these examples shown herein, as long as it conforms to the principles and features disclosed herein.

## Claims

1. A bladder perfusion pharmaceutical composition, comprising: an immunological adjuvant or a soluble salt thereof, and a chemical drug capable of causing immunogenic cell death.

2. The bladder perfusion pharmaceutical composition according to claim 1, wherein the chemical drug capable of causing immunogenic cell death comprises: an anthracycline-based chemotherapeutic drug, a platinum-based chemotherapeutic drug, fluorouracil, or gemcitabine.

3. The bladder perfusion pharmaceutical composition according to claim 2, wherein the anthracycline-based chemotherapeutic drug comprises epirubicin or a soluble salt thereof, pirarubicin or a soluble salt thereof, mitoxantrone or a soluble salt thereof, doxorubicin or a soluble salt thereof, aclarubicin or a soluble salt thereof, and idarubicin or a soluble salt thereof.

4. The bladder perfusion pharmaceutical composition according to claim 3, wherein the soluble salt is hydrochloride.

5. The bladder perfusion pharmaceutical composition according to any one of claims 2-4, wherein the anthracycline-based chemotherapeutic drug comprises doxorubicin hydrochloride, epirubicin hydrochloride, pirarubicin hydrochloride, and mitoxantrone hydrochloride.

6. The bladder perfusion pharmaceutical composition according to any one of claims 2-5, wherein the platinum-based chemotherapeutic drug comprises nedaplatin, carboplatin, lobaplatin, or oxaliplatin.

7. The bladder perfusion pharmaceutical composition according to any one of claims 1-6, wherein the immunological adjuvant or the soluble salt thereof comprises a soluble salt of the immunological adjuvant.

8. The bladder perfusion pharmaceutical composition according to any one of claims 1-7, wherein the immunological adjuvant or the soluble salt thereof comprises an imidazoquinoline-based immunological adjuvant or a soluble salt thereof.

9. The bladder perfusion pharmaceutical composition according to claim 8, wherein the imidazoquinoline-based immunological adjuvant comprises imiquimod and a derivative thereof, or resiquimod and a derivative thereof, or a soluble salt of imiquimod and the derivative thereof, or a soluble salt of resiquimod and the derivative thereof.

10. The bladder perfusion pharmaceutical composition according to any one of claims 7-9, wherein the soluble salt of the immunological adjuvant comprises at least one of imiquimod R837 hydrochloride, resiquimod R848 hydrochloride or other pharmaceutically acceptable salts, CpG, polyIC, polyICLC and a STING stimulator.

11. The bladder perfusion pharmaceutical composition according to any one of claims 1-10, wherein the mass ratio of the immunological adjuvant or the soluble salt thereof to the chemotherapy drug capable of causing immunogenic cell death is 1:100 to 6:1.

12. The bladder perfusion pharmaceutical composition according to any one of claims 10-11, wherein the mass ratio of the imiquimod R837 hydrochloride to the chemical drug capable of causing immunogenic cell death is 1:20 to 1:1, and the chemical drug capable of causing immunogenic cell death is fluorouracil or gemcitabine.

13. The bladder perfusion pharmaceutical composition according to any one of claims 10-11, wherein the mass ratio of the imiquimod R837 hydrochloride to the chemical drug capable of causing immunogenic cell death is 1:1 to 6:1, wherein the chemical drug comprises an anthracycline-based chemotherapeutic drug or a platinum-based chemotherapeutic drug, and the anthracycline-based chemotherapeutic drug comprises doxorubicin hydrochloride, epirubicin hydrochloride, pirarubicin hydrochloride, and mitoxantrone hydrochloride; and the platinum-based chemotherapeutic drug comprises nedaplatin, carboplatin, lobaplatin, or oxaliplatin.

14. The bladder perfusion pharmaceutical composition according to any one of claims 10-11, wherein the mass ratio of the resiquimod R848 hydrochloride to the chemical drug capable of causing immunogenic cell death is 1:20 to 1:1, wherein the chemical drug capable of causing immunogenic cell death comprises an anthracycline-based chemotherapeutic drug or a platinum-based chemotherapeutic drug, and the anthracycline-based chemotherapeutic drug comprises doxorubicin hydrochloride, epirubicin hydrochloride, pirarubicin hydrochloride, or mitoxantrone hydrochloride; and the platinum-based chemotherapeutic drug comprises nedaplatin, carboplatin, lobaplatin, or oxaliplatin.

15. The bladder perfusion pharmaceutical composition according to any one of claims 10-11, wherein the mass ratio of the resiquimod R848 hydrochloride to the chemical drug capable of causing immunogenic cell death is 1:100 to 1:10, wherein the chemical drug capable of causing immunogenic cell death comprises fluorouracil or gemcitabine.

16. The bladder perfusion pharmaceutical composition according to any one of claims 1-15, wherein the concentration of the immunological adjuvant ranges from 0.5 mg/mL to 30 mg/mL.

17. The bladder perfusion pharmaceutical composition according to any one of claims 1-16, further comprising a pH regulator.

18. The bladder perfusion pharmaceutical composition according to claim 17, wherein after the bladder perfusion pharmaceutical composition is reconstituted, the pH regulator enables the pH of the reconstituted solution to be 3.8 to 5.5 when the concentration of the anthracycline-based chemotherapy drug is 1 to 5 mg/mL.

19. The bladder perfusion pharmaceutical composition according to any one of claims 1-18, further comprising a lyoprotectant.

20. The bladder perfusion pharmaceutical composition according to claim 19, wherein the lyoprotectant comprises at least one of sucrose, lactose, mannitol, and cyclodextrin.

21. The bladder perfusion pharmaceutical composition according to claim 20, wherein the lyoprotectant is lactose.

22. The bladder perfusion pharmaceutical composition according to any one of claims 1-21, which is a lyophilized powder formulation.

23. The bladder perfusion pharmaceutical composition according to any one of claims 1-22, wherein the mass ratio of the anthracycline-based chemotherapy drug to the immunological adjuvant is 1:0.1 to 1:10.

24. The bladder perfusion pharmaceutical composition according to claim 23, wherein the mass ratio of the anthracycline-based chemical drug to imiquimod and the derivative thereof is 1:1 to 1:10.

25. The bladder perfusion pharmaceutical composition according to claim 23, wherein the mass ratio of the anthracycline-based chemical drug to the soluble salt of imiquimod and the derivative thereof is 1:1 to 1:10.

26. The bladder perfusion pharmaceutical composition according to claim 23, wherein the mass ratio of the anthracycline-based chemical drug to resiquimod is 1:0.1 to 1:5.

27. The bladder perfusion pharmaceutical composition according to claim 23, wherein the mass ratio of the anthracycline-based chemical drug to the soluble salt of resiquimod and the derivative thereof is 1:0.1 to 1:5.

28. The bladder perfusion pharmaceutical composition according to any one of claims 1-27, which comprises: epirubicin or a soluble salt thereof, imiquimod or a soluble salt thereof, and a pH regulator.

29. The bladder perfusion pharmaceutical composition according to claim 28, wherein the bladder perfusion pharmaceutical composition is a lyophilized powder dosage form.

30. The bladder perfusion pharmaceutical composition according to claim 28, further comprising a lyoprotectant.

31. The bladder perfusion pharmaceutical composition according to any one of claims 29-30, wherein the pH of the lyophilized powder dosage form after reconstitution is 3.8 to 5.5 when the concentration of epirubicin is 1 to 5 mg/mL.

32. The bladder perfusion pharmaceutical composition according to any one of claims 29-31, wherein the pH of the lyophilized powder dosage form after reconstitution is 4.0 to 5.0 when the concentration of epirubicin is 1 to 5 mg/mL.

33. The bladder perfusion pharmaceutical composition according to any one of claims 29-32, wherein the pH of the lyophilized powder dosage form after reconstitution is 4.0 to 4.2, 4.2 to 4.5 or 4.5 to 5.0 when the concentration of epirubicin is 1 to 5 mg/mL.

34. The bladder perfusion pharmaceutical composition according to any one of claims 28-33, wherein the mass ratio of the epirubicin or the hydrochloride thereof to imiquimod or the soluble salt thereof is 1:1 to 1:10.

35. The bladder perfusion pharmaceutical composition according to any one of claims 28-34, wherein the mass ratio of the epirubicin or the hydrochloride thereof to imiquimod or the soluble salt thereof is 1:2 to 1:4.

36. The bladder perfusion pharmaceutical composition according to any one of claims 30-35, wherein the lyoprotectant comprises sucrose, lactose, mannitol, and cyclodextrin.

37. The bladder perfusion pharmaceutical composition according to any one of claims 30-36, wherein the lyoprotectant is lactose.

38. The bladder perfusion pharmaceutical composition according to any one of claims 30-37, wherein the mass fraction of the lyoprotectant in the lyophilized powder formulation is 65% to 96%.

39. The bladder perfusion pharmaceutical composition according to any one of claims 30-38, wherein the content of the lyoprotectant in the lyophilized powder formulation is 80% to 94%.

40. The bladder perfusion pharmaceutical composition according to any one of claims 1-39, further comprising a mucosal penetration enhancer.

41. The bladder perfusion pharmaceutical composition according to claim 40, wherein the mucosal penetration enhancer comprises at least one of azone, hyaluronidase, lauryl alcohol, and oleic acid.

42. A method for preparing a bladder perfusion pharmaceutical composition, comprising the following steps:
S1: dissolving an immunological adjuvant with a dilute acid to obtain a dilute acid salt solution of the immunological adjuvant; S2: dissolving a chemical drug capable of causing immunogenic cell death with the dilute acid salt solution obtained in S1 to obtain a mixed solution; and S3: adding a pH regulator into the mixed solution in S1, and controlling the pH between 2.0 and 5.5.

43. The preparation method according to claim 42, wherein S3 further comprises the following steps:
S31: adding a lyoprotectant into the mixed solution; and/or
S32: adding a mucosal penetration enhancer into the mixed solution.

44. The preparation method according to claim 42, further comprising step S4: lyophilizing the final solution.

45. The preparation method according to any one of claims 42-44, wherein the pH is controlled between 3.8 and 5.5 in the step S3.

46. The preparation method according to any one of claims 42-45, wherein the chemical drug capable of causing immunogenic cell death comprises an anthracycline-based chemical drug, and the final concentration of the anthracycline-based chemical drug in step S3 is 1 to 5 mg/mL.

47. A method for preparing a bladder perfusion pharmaceutical composition, comprising the following steps: S1: adding immunological adjuvants CpG, polyIC, polyICLC, and a water soluble STING stimulator as well as a chemical drug capable of causing immunogenic cell death into water for injection, and uniformly mixing until dissolution; and S2: adding a suitable lyoprotectant into the solution in S1, and lyophilizing the solution.

48. Use of a bladder perfusion pharmaceutical composition according to any one of claims 1-41 in a preparation of a bladder perfusion formulation.
